# EUROPEAN PATENT APPLICATION

(11) **EP 1 533 378 A2**
(43) Date of publication of application: **25.05.2005**
(21) Application number: 05001468.7
(22) Date of filing: 06.04.2000
(51) Int. Cl.: C12N 15/12, C07K 14/47, C12N 15/11, C07K 16/16, C12N 5/10, C12N 5/12, G01N 33/50, C12Q 1/68

(54) **Tyrosine kinase substrate protein Tks7**

(30) Priority: 09.04.1999 US 128492 P
(62) Divisional of application: 00921848.8
(71) Applicant: Sugen, Inc., South San Francisco, CA 94080 (US)
(72) Inventor: Phan, Huan, Belmont, CA 94002 (US); Courtneidge, Sara A., Burlingame, CA 94010 (US)
(74) Representative: Viering, Jentschura & Partner

(57) **Abstract**

The present invention relates to novel Tks 107, Tks 113, Tsk 118 and Tks 202 polypeptides, nucleotide sequences encoding polypeptides, as well as various products and methods useful for the diagnosis and treatment of various related diseases and conditions.

## Description

### FIELD OF THE INVENTION

The present invention relates to novel polypeptides, nucleotide sequences encoding the novel polypeptides, as well as various products and methods useful for the diagnosis and treatment of various diseases and conditions.

### BACKGROUND OF THE INVENTION

The following description of the background of the invention is provided to aid in understanding the invention, but is not admitted to be or to describe prior art to the invention.

Cellular signal transduction is a fundamental mechanism whereby external stimuli that regulate diverse cellular processes are relayed to the interior of cells. One of the key biochemical mechanisms of signal transduction involves the reversible phosphorylation through kinases of proteins, which enables regulation of the activity of mature proteins by altering their structure and function.

The best characterized protein kinases in eukaryotes phosphorylate proteins on the hydroxyl substituent of serine, threonine and tyrosine residues. These kinases largely fall into two groups, those specific for phosphorylating serines and threonines, and those specific for phosphorylating tyrosines. Some kinases, referred to as "dual specificity" kinases, are able to phosphorylate on tyrosine as well as serine/threonine residues.

Many kinases are involved in regulatory cascades wherein their substrates may include other kinases whose activities are regulated by their phosphorylation state. Ultimately the activity of some downstream effector is modulated by phosphorylation resulting from activation of such a pathway.

Non-receptor tyrosine kinases may be recruited to the plasma membrane where they mediate cellular signaling by cell surface receptors lacking intrinsic protein tyrosine kinase activities. For instance, members of the Src family of protein tyrosine kinases are activated in response to stimulation of growth factor receptors and G-protein coupled receptors, as well as many other extracellular stimuli (Thomas, *et al.,* 1997. Annu. Rev. Cell. Dev. Biol. 13:513-609).

Src family kinases have been found associated with coated membrane regions in platelets (Stenberg, *et al.,* 1997. Blood 89:2384-93). Src copurifies with synaptic vesicles in PC12 cells (Linstedt, *et* al., 1992. J. Cell Biol. 117:1077-84). Src associates with and phosphorylates several proteins involved in membrane trafficking, such as the neuronal synaptic vesicle associated protein synapsin I, synaptophysin and synaptogyrin (Barnekow, *et al.,* 1990. Oncogene 5:1019-24; Foster-Barber, *et al.,* 1998. Proc. Natl. Acad. Sci. USA 95:4673-7; Janz, *et al.,* 1998. J. Biol. Chem. 273:2851-7).

Small GTPases represent a large family of proteins that act as molecular switches that control diverse biological functions, including cell proliferation and differentiation, cytoskeletal organization, protein transport, cell cycle and free radical production (Bourne, H.R. et. al., Nature, 348(6297):125-32, 1990. Bourne, H.R. et. al., Nature, 349(6305):117-27, 1991). That GTPases modulate these central cellular pathways suggest that both GTPases and their regulators are of critical importance.

The dbl homology region (DH domain) is a region of approximately 250 amino acids initially found in the Dbl and Cdc24 proteins. Many proteins have been found to contain DH domains. Many DH containing proteins exhibit cellular transformation activity upon N-terminal truncation. (Reviewed in Quilliam, L.A., et. al. BioEssays, 17:395-404, 1995. Whitehead, I.P., et. al., Biochemica et Biophysica Acta, 1332:F1-F23, 1997. Cerione, R.A. and Zheng, Y. Curr. Opin. Cell. Biol. 8:216-222, 1996).

The DH/PH modules of many Dbl family proteins have been shown to regulate the activity of various Rho sub-family small GTPases by serving as Guanine Exchange Factors (GEF) (Whitehead, I.P., et. al., Biochimica et Biophysica Acta, 1332:F1-F23, 1997. Cerione, R.A. and Zheng, Y. Curr. Opin. Cell Biol. 8:216-222, 1996). Many Rho family GTPases have been shown to regulate the assembly of actin structures and may also regulate gene transcription through various kinase pathways (Hall, A. Science, 279:509-514, 1998).

### SUMMARY OF THE INVENTION

The present invention relates to novel substrates of the cytoplasmic tyrosine kinase Src, Tks 107, Tks 113, Tks 118 and Tks 202.

A first aspect of the invention features an isolated, enriched or purified nucleic acid molecule encoding a polypeptide selected from the group consisting of Tks 107, Tks 113, Tks 118 and Tks 202.

By "isolated" in reference to nucleic acid is meant a polymer of nucleotides conjugated to each other, including DNA and RNA, that is isolated from a natural source or that is synthesized. The isolated nucleic acid of the present invention is unique in the sense that it is not found in a pure or separated state in nature. Use of the term "isolated" indicates that a naturally occurring sequence has been removed from its normal cellular (i.e., chromosomal) environment. Thus, the sequence may be in a cell-free solution or placed in a different cellular environment. The term does not imply that the sequence is the only nucleotide chain present, but that it is essentially free (about 90 - 95% pure at least) of non-nucleotide material naturally associated with it, and thus is distinguished from isolated chromosomes.

By the use of the term "enriched" in reference to nucleic acid is meant that the specific DNA or RNA sequence constitutes a significantly higher fraction (2 - 5 fold) of the total DNA or RNA present in the cells or solution of interest than in normal or diseased cells or in the cells from which the sequence was taken. This could be caused by a person by preferential reduction in the amount of other DNA or RNA present or by a preferential increase in the amount of the specific DNA or RNA sequence or by a combination of the two. However, it should be noted that enriched does not imply that there are no other DNA or RNA sequences present, just that the relative amount of the sequence of interest has been significantly increased. The term "significant" is used to indicate that the level of increase is useful to the person making such an increase, and generally means an increase relative to other nucleic acids of about at least 2 fold, more preferably at least 5 to 10 fold or even more. The term also does not imply that there is no DNA or RNA from other sources. The other source DNA may, for example, comprise DNA from a yeast or bacterial genome, or a cloning vector such as pUC19. This term distinguishes from naturally occurring events, such as viral infection, or tumor type growths, in which the level of one mRNA may be naturally increased relative to other species of mRNA. That is, the term is meant to cover only those situations in which a person has intervened to elevate the proportion of the desired nucleic acid.

It is also advantageous for some purposes that a nucleotide sequence be in purified form. The term "purified" in reference to nucleic acid does not require absolute purity (such as a homogeneous preparation). Instead, it represents an indication that the sequence is relatively more pure than in the natural environment (compared to the natural level this level should be at least 2-5 fold greater, *e.g.,* in terms of mg/mL). Individual clones isolated from a cDNA library may be purified to electrophoretic homogeneity. The claimed DNA molecules obtained from these clones could be obtained directly from total DNA or from total RNA. The cDNA clones are not naturally occurring, but rather are preferably obtained via manipulation of a partially purified naturally occurring substance (messenger RNA). The construction of a cDNA library from mRNA involves the creation of a synthetic substance (cDNA) and pure individual cDNA clones can be isolated from the synthetic library by clonal selection of the cells carrying the cDNA library. Thus, the process which includes the construction of a cDNA library from mRNA and isolation of distinct cDNA clones yields an approximately 10⁶-fold purification of the native message. Thus, purification of at least one order of magnitude, preferably two or three orders, and more preferably four or five orders of magnitude is expressly contemplated.

By a polypeptide is meant at least 108, 93, 60 or 203 or more contiguous amino acids set forth in the amino acid sequence of SEQ ID NO:5, SEQ ID NO:6, SEQ ID NO:7 or SEQ ID NO:8, respectively, or the corresponding full-length amino acid sequence; or a functional derivative thereof as described herein. For sequences for which the full-length sequence is not given, the remaining sequences can be determined using methods well-known to those in the art and are intended to be included in the invention. The polypeptide can be encoded by a full-length nucleic acid sequence or any portion of the full-length nucleic acid sequence, so long as a functional activity of the polypeptide is retained.

The amino acid sequence will be substantially similar to the sequence shown in SEQ ID NO:5, SEQ ID NO:6, SEQ ID NO:7 or SEQ ID NO:8, or the corresponding full-length amino acid sequence, or fragments thereof. A sequence that is substantially similar to the sequence of SEQ ID NO:5, SEQ ID NO:6, SEQ ID NO:7 or SEQ ID NO:8 will preferably have at least 90% identity (more preferably at least 95% and most preferably 99-100%) to the respective sequence.

By "identity" is meant a property of sequences that measures their similarity or relationship. Identity is measured by dividing the number of identical residues by the total number of residues and gaps and multiplying the product by 100. "Gaps" are spaces in an alignment that are the result of additons or deletions of amino acids. Thus, two copies of exactly the same sequence have 100% identity, but sequences that are less highly conserved, and have deletions, additions, or replacements, may have a lower degree of identity. Those skilled in the art will recognize that several computer programs are available for determining sequence identity using standard parameters as default settings. Examples of such programs are Gapped BLAST or PSI-BLAST (Altschul, *et al.* (1997) Nucleic Acids Res. 25:3389-3402), BLAST (Altschul, *et al.* (1990) J. Mol. Biol. 215:403-410), and Smith-Waterman (Smith, *et al.* (1981) J. Mol. Biol. 147:195-197). Preferably, the default settings of these programs will be employed, but those skilled in the art recognize whether these settings need to be changed and know how to make the changes.

In preferred embodiments, the invention features isolated, enriched or purified nucleic acid molecules encoding a polypeptide comprising a nucleotide sequence that: (a) encodes a polypeptide having the amino acid sequence set forth in SEQ ID NO:5, SEQ ID NO:6, SEQ ID NO:7 or SEQ ID NO:8; (b) is the complement of the nucleotide sequence of (a); (c) hybridizes under highly stringent conditions to the nucleotide molecule of (a) or (b) and encodes a naturally occurring polypeptide; (d) differs from a polypeptide having the amino acid sequence of SEQ ID NO:5, SEQ ID NO:6, SEQ ID NO:7 or SEQ ID NO:8 by lacking one or more, but not all, of the following segments of amino acid residues: 1-1082, 1083-1257, 1258-1264, 1265-1372 or 1373-1519 of SEQ ID NO:5; 1-383 or 384-476 of SEQ ID NO:6; 1-227, 228-371 or 372-431 or SEQ ID NO:7; or 1-629 or 630-832 or SEQ ID NO:8; (e) is the complement of the nucleotide sequence of (d); (f) encodes a polypeptide having the amino acid sequence set forth in amino acid residues: 1-1082, 1083-1257, 1258-1264, 1265-1372 or 1373-1519 of SEQ ID NO:5; 1-383 or 384-476 of SEQ ID NO:6; 1-227, 228-371 or 372-431 or SEQ ID NO:7; or 1-629 or 630-832 or SEQ ID NO:8; (g) is the complement of the nucleotide sequence of (f); (h) differs from a polypeptide having the amino acid sequence set forth in SEQ ID NO:5, SEQ ID NO:6, SEQ ID NO:7 or SEQ ID NO:8 by lacking one or more of the domains selected from the group consisting of an N-terminal domain, a DH domain, a C-terminal domain, a PH domain, a spacer region, an SH3 domain, a GTPase domain and a proline-rich region; or (i) is the complement of the nucleotide sequence of (h).

The term "complement" refers to two nucleotides that can form multiple favorable interactions with one another. For example, adenine is complementary to thymine as they can form two hydrogen bonds. Similarly, guanine and cytosine are complementary since they can form three hydrogen bonds. A nucleotide sequence is the complement of another nucleotide sequence if all of the nucleotides of the first sequence are complementary to all of the nucleotides of the second sequence.

The term "domain" refers to a region of a polypeptide which contains a particular function. For instance, N-terminal or C-terminal domains of signal transduction proteins can serve functions including, but not limited to, binding molecules that localize the signal transduction molecule to different regions of the cell or binding other signaling molecules directly responsible for propagating a particular cellular signal. Some domains can be expressed separately from the rest of the protein and function by themselves, while others must remain part of the intact protein to retain function. The latter are termed functional regions of proteins and also relate to domains.

The term "N-terminal domain" refers to the extracatalytic region located between the initiator methionine and the subsequent domain of the protein. The N-terminal domain can be identified following a Smith-Waterman alignment of the protein sequence against the non-redundant protein database to define the N-terminal boundary of the catalytic domain. Depending on its length, the N-terminal domain may or may not play a regulatory role in kinase function. An example of a protein kinase whose N-terminal domain has been shown to play a regulatory role is p65PAK, which contains a CRIB motif used for Cdc42 and rac binding (Burbelo, P.D. *et al.* (1995) J. Biol. Chem. 270, 29071-29074).

The N-terminal domain spans amino acid residues 1-2082 of SEQ ID NO:5, 1-383 of SEQ ID NO:6, 1-227 of SEQ ID NO: 7 and 1-629 of SEQ ID NO:8.

The term "substrate" as used herein refers to a molecule phosphorylated by a protein of the invention.

The term "C-terminal domain" refers to the region located between the last (located closest to the C-terminus) functional domain and the carboxy-terminal amino acid residue of the preceding domain. By "functional" domain is meant any region of the polypeptide that may play a regulatory or catalytic role as predicted from amino acid sequence homology to other proteins or by the presence of amino acid sequences that may give rise to specifc structural conformations. The C-terminal domain can be identified by using a Smith-Waterman alignment of the protein sequence against the non-redundant protein database to define the C-terminal boundary of the catalytic domain or of any functional C-terminal extracatalytic domain.

The C-terminal domain spans amino acid residues 1373-1519 of SEQ ID NO:5.

The term "dbl homology domain (DH domain)" refers to a domain composed essentially of α-helices, several of which correspond to previously defined conserved regions (Soisson, S.M., et. al., Cell, 95:259-268), as identified by a hidden Markov model (http://hmmer.wustl.edu).

The DH domain spans amino acid residues 1083-1257 of SEQ ID NO:5.

By "PH domain" is meant a polypeptide having homology to an approximately 100 amino acid region of pleckstrin. The total number of proposed PH domains now exceeds 70. Recent structural studies have demonstrated that PH domains are distinct structural modules. The fold is best described as a seven-stranded sandwich of two orthogonal b sheets that is closed at one corner by a C-terminal helix. A polarization of the domain is evident, with the three most variable loops forming a positively charged surface at the corner of the sandwich opposite from that closed off by the helix. Ferguson, K.M. *et al.,* Cell, 79, 199-209, 1994, incorporated herein by reference in its entirety, including any drawings. Examples of various PH domains are provided in Musacchio, A., *et al*., TIBS, 18:343-348, 1993 and Gibson, T.J., *et al.,* TIBS, 19:349-353, 1994, both of which are incorporated herein by reference in their entirety, including any drawings. Other PH domains may be identified using the sequence alignment techniques and three dimensional structure comparisons described in those publications. PH domains include those in serine/threonine as well as tyrosine kinases; regulators of small GTP-binding proteins; cytoskeletal proteins; and putative signaling adapter molecules. PH domains are also those from dynamin, proteins involved in cellular membrane transport and phospholipase C isoforms. The cloning and sequence of multiple forms of phospholipase C is described in Suh, *et al.,* Cell, 54:161-169, 1988, icorporated herein by reference in its entirety, including any drawings.

The PH domain spans amino acids 1265-1372 of SEQ ID NO:5.

The terms "SH3" domain" and GTPase domain are well-known terms and the meaning is defined as known to one skilled in the art.

The SH3 domain spans amino acids 372-431 of SEQ ID NO:9. The GTPase domain spans amino acids 630-832 of SEQ ID NO:8.

The term "signal transduction pathway" refers to the molecules that propagate an extracellular signal through the cell membrane to become an intracellular signal. This signal can then stimulate a cellular response. The polypeptide molecules involved in signal transduction processes are typically receptor and non-receptor protein tyrosine kinases, receptor and non-receptor protein phosphatases, SRC homology 2 and 3 domains, phosphotyrosine binding proteins (SRC homology 2 (SH2) and phosphotyrosine binding (PTB and PH) domain containing proteins), proline-rich binding proteins (SH3 domain containing proteins), nucleotide exchange factors, and transcription factors.

The term "proline-rich region" as used herein, refers to a region of a protein whose proline content over a given amino acid length is higher than the average content of this amino acid found in proteins (i.e., >10%). Proline-rich regions are easily discernable by visual inspection of amino acid sequences and quantitated by standard computer sequence analysis programs such as the DNAStar program EditSeq. Proline-rich regions have been demonstrated to participate in regulatory protein -protein interactions. *(see for example* Galisteo, M.L. *et al.* (1996) J. Biol. Chem. 271:20997-21000 and Sudol, M. (1996) Prog. Biochys. Mol. Bio. 65:113-132).

The proline-rich region spans amino acid residues 384-476 of SEQ ID NO:5.

The term "spacer region" as used herein, refers to a region of the protein located between predicted functional domains. The spacer region has no detectable homology to any amino acid sequence in the database and can be identified by using a Smith-Waterman alignment of the protein sequence against the non-redundant protein database to define the C- and N-terminal boundaries of the flanking functional domains. Spacer regions may or may not play a fundamental role in protein kinase function. Precedence for the regulatory role of spacer regions in kinase function is provided.by the role of the src kinase spacer in inter-domain interactions (Xu, W. *et al.* (1997) Nature 385:595-602).

The spacer region spans amino acid residues 1258-1264 of SEQ ID NO:5.

Various low or high stringency hybridization conditions may be used depending upon the specificity and selectivity desired. These conditions are well-known to those skilled in the art. Under stringent hybridization conditions only highly complementary nucleic acid sequences hybridize. Preferably, such conditions prevent hybridization of nucleic acids having more than 1 or 2 mismatches out of 20 contiguous nucleotides, more preferably, such conditions prevent hybridization of nucleic acids having more than 1 or 2 mismatches out of 50 contiguous nucleotides, most preferably, such conditions prevent hybridization of nucleic acids having more than 1 or 2 mismatches out of 100 contiguous nucleotides. In some instances, the conditions may prevent hybridization of nucleic acids having more than 5 mismatches in the full-length sequence.

By stringent hybridization assay conditions is meant hybridization assay conditions at least as stringent as the following: hybridization in 50% formamide, 5X SSC, 50 mM NaH2PO4, pH 6.8, 0.5% SDS, 0.1 mg/mL sonicated salmon sperm DNA, and 5X Denhart solution at 42°C overnight; washing with 2X SSC, 0.1% SDS at 45°C; and washing with 0.2X SSC, 0.1% SDS at 45°C. More stringent conditions include 0.1XSSC, 0.05% SDS and 55°C for the second wash. Under some of the most stringent hybridization assay conditions, the second wash can also be done with 0.1XSSC at a temperature up to 70°C (Berger *et al.* (1987) Guide to Molecular Cloning Techniques pg 421, hereby incorporated by reference herein in its entirety including any figures, tables or drawings.). However, other applications may require the use of conditions falling between these sets of conditions. Methods of determining the conditions required to achieve desired hybridizations are well known to those with ordinary skill in the art and are based on several factors, including but not limited to, the sequences to be hybridized and the samples to be tested. Washing conditions of lower stringency frequently utilize a lower temperature during the washing steps, such as 65°C, 60°C, 55°C, 50°C or 42°C.

In other preferred embodiments, the invention features isolated, enriched or purified nucleic acid molecules encoding polypeptides, further comprising a vector or promoter effective to initiate transcription in a host cell. The invention also features a recombinant nucleic acid, preferably in a cell or an organism. The recombinant nucleic acid may contain a sequence set forth in SEQ ID NO:1, SEQ ID NO:2, SEQ ID NO:3 or SEQ ID NO:4, a nucleic aicd encoding the polypeptide set forth in SEQ ID NO:5, SEQ ID NO:6, SEQ ID NO:7, or SEQ ID NO:8, or a functional derivative thereof and a vector or a promoter effective to initiate transcription in a host cell. The recombinant nucleic acid can alternatively contain a transcriptional initiation region functional in a cell, a sequence complementary to an RNA sequence encoding a polypeptide and a transcriptional termination region functional in a cell.

The term "vector" relates to a single or double-stranded circular nucleic acid molecule that can be transfected into cells and replicated within or independently of a cell genome. A circular double-stranded nucleic acid molecule can be cut and thereby linearized upon treatment with restriction enzymes. An assortment of nucleic acid vectors, restriction enzymes and the knowledge of the nucleotide sequences cut by restriction enzymes are readily available to those skilled in the art. A nucleic acid molecule encoding a kinase can be inserted into a vector by cutting the vector with restriction enzymes and ligating the two pieces together.

The term "transfecting" defines a number of methods to insert a nucleic acid vector or other nucleic acid molecules into a cellular organism. These methods involve a variety of techniques, such as treating the cells with high concentrations of salt, an electric field, detergent or DMSO to render the outer membrane or wall of the cells permeable to nucleic acid molecules of interest or use of various viral transduction strategies.

The term "promoter" as used herein, refers to nucleic acid sequence(s) needed for gene sequence expression. Promoter regions vary from organism to organism, but are well known to persons skilled in the art for different organisms. For example, in prokaryotes, the promoter region contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence, CAAT sequence and the like.

In preferred embodiments, the isolated nucleic acid comprises, consists essentially of, or consists of a nucleic acid sequence set forth in SEQ ID NO:1, SEQ ID NO:2, SEQ ID NO:3 or SEQ ID NO:4, or the corresponding full-length sequence, encodes the amino acid sequence of SEQ ID NO:5, SEQ ID NO:6, SEQ ID NO:7 or SEQ ID NO:8, or the corresponding full-length amino acid sequence, a functional derivative thereof, or at least 108, 93, 60 or 203 contiguous amino acids of SEQ ID NO:5, SEQ ID NO:6, or SEQ ID NO:7 or SEQ ID NO:8, respectively, or of the corresponding full-length amino acid sequence. The nucleic acid may be isolated from a natural source by cDNA cloning or by subtractive hybridization. The natural source may be mammalian, preferably human, blood, semen, or tissue, and the nucleic acid may be synthesized by the triester method or by using an automated DNA synthesizer.

The term "mammal" refers preferably to such organisms as mice, rats, rabbits, guinea pigs, sheep, and goats, more preferably to cats, dogs, monkeys, and apes, and most preferably to humans.

In yet other preferred embodiments, the nucleic acid is a conserved or unique region, for example those useful for: the design of hybridization probes to facilitate identification and cloning of additional polypeptides, the design of PCR probes to facilitate cloning of additional polypeptides, obtaining antibodies to polypeptide regions, and designing antisense oligonucleotides.

By "conserved nucleic acid regions" are meant regions present on two or more nucleic acids encoding a polypeptide, to which a particular nucleic acid sequence can hybridize under lower stringency conditions. Examples of lower stringency conditions suitable for screening for nucleic acid encoding kinase polypeptides are provided in Berger *et al.* (1987) Guide to Molecular Cloning Techniques pg 421 (hereby incorporated by reference herein in its entirety including any figures, tables or drawings). Preferably, conserved regions differ by no more than 7 out of 20 nucleotides.

By "unique nucleic acid region" is meant a sequence present in a nucleic acid coding for a polypeptide that is not present in a sequence coding for any other naturally occurring polypeptide. Such regions preferably encode 108, 93, 60 or 203 or more contiguous amino acids set forth in the amino acid sequence of SEQ ID NO:5, SEQ ID NO:6, or SEQ ID NO:7 or SEQ ID NO:8, respectively, or the corresponding full-length amino acid sequence or functional derivatives thereof. In particular, a unique nucleic acid region is preferably of mammalian origin.

Another aspect of the invention features a nucleic acid probe for the detection of nucleic acid encoding a polypeptide in a sample, wherein said polypeptide is selected from the group consisting of Tks 107, Tks 113, Tks 118 and Tks 202. The nucleic acid probe may encode a polypeptide that is a fragment of the protein encoded by the amino acid sequence set forth in SEQ ID NO:5, SEQ ID NO:6, SEQ ID NO:7 or SEQ ID NO:8, or the corresponding full-length amino acid sequences or a complement thereof. The nucleic acid probe contains a nucleotide base sequence that will hybridize to a sequence set forth in SEQ ID NO:1, SEQ ID NO:2, SEQ ID NO:3 or SEQ ID NO:4, or the corresponding full-length sequence, or a functional derivative thereof.

Methods for using the probes include detecting the presence or amount of RNA in a sample by contacting the sample with a nucleic acid probe under conditions such that hybridization occurs and detecting the presence or amount of the probe bound to RNA. The nucleic acid duplex formed between the probe and a nucleic acid sequence coding for a polypeptide may be used in the identification of the sequence of the nucleic acid detected (Nelson *et al.,* in Nonisotopic DNA Probe Techniques, Academic Press, San Diego, Kricka, ed., p. 275, 1992, hereby incorporated by reference herein in its entirety, including any drawings, figures, or tables). Kits for performing such methods may be constructed to include a container means having disposed therein a nucleic acid probe.

In another aspect, the invention describes recombinant DNA molecules or a cell or tissue comprising a recombinant nucleic acid molecule encoding a polypeptide selected from the group consisting of Tks 107, Tks 113, Tks 118 and Tks 202. In such cells, the nucleic acid may be under the control of the genomic regulatory elements, or may be under the control of exogenous regulatory elements including an exogenous promoter. By "exogenous" it is meant a promoter that is not normally coupled in vivo transcriptionally to the coding sequence for the polypeptides.

The polypeptide is preferably a fragment of the protein encoded by the amino acid sequence set forth in SEQ ID NO:5, SEQ ID NO:6, SEQ ID NO:7 or SEQ ID NO:8 or full-length sequence thereof. By "fragment," is meant an amino acid sequence present in a polypeptide. Preferably, such a sequence comprises at least 108, 93, 60 or 203 contiguous amino acids of SEQ ID NO:5, SEQ ID NO:6, SEQ ID NO:7 or SEQ ID NO:8, respectively.

In another aspect, the invention features an isolated, enriched or purified polypeptide selected from the group consisting of Tks 107, Tks 113, Tks 118 and Tks 202.

By "isolated" in reference to a polypeptide is meant a polymer of amino acids (2 or more amino acids) conjugated to each other, including polypeptides that are isolated from a natural source or that are synthesized. The isolated polypeptides of the present invention are unique in the sense that they are not found in a pure or separated state in nature. Use of the term "isolated" indicates that a naturally occurring sequence has been removed from its normal cellular environment. Thus, the sequence may be in a cell-free solution or placed in a different cellular environment. The term does not imply that the sequence is the only amino acid chain present, but that it is essentially free (about 90 - 95% pure at least) of non-amino acid material naturally associated with it.

By the use of the term "enriched" in reference to a polypeptide is meant that the specific amino acid sequence constitutes a significantly higher fraction (2 - 5 fold) of the total amino acid sequences present in the cells or solution of interest than in normal or diseased cells or in the cells from which the sequence was taken. This could be caused by a person by preferential reduction in the amount of other amino acid sequences present, or by a preferential increase in the amount of the specific amino acid sequence of interest, or by a combination of the two. However, it should be noted that enriched does not imply that there are no other amino acid sequences present, just that the relative amount of the sequence of interest has been significantly increased. The term significant here is used to indicate that the level of increase is useful to the person making such an increase, and generally means an increase relative to other amino acid sequences of about at least 2-fold, more preferably at least 5- to 10-fold or even more. The term also does not imply that there is no amino acid sequence from other sources. The other source of amino acid sequences may, for example, comprise amino acid sequence encoded by a yeast or bacterial genome, or a cloning vector such as pUC19. The term is meant to cover only those situations in which one has intervened to increase the proportion of the desired amino acid sequence.

It is also advantageous for some purposes that an amino acid sequence be in purified form. The term "purified" in reference to a polypeptide does not require absolute purity (such as a homogeneous preparation); instead, it represents an indication that the sequence is relatively purer than in the natural environment. Compared to the natural level this level should be at least 2-5 fold greater (*e.g.,* in terms of mg/mL). Purification of at least one order of magnitude, preferably two or three orders, and more preferably four or five orders of magnitude is expressly contemplated. The substance is preferably free of contamination at a functionally significant level, for example 90%, 95%, or 99% pure.

In preferred embodiments, the polypeptide is a fragment of the protein encoded by the amino acid sequence set forth in SEQ ID NO:5, SEQ ID NO:6, SEQ ID NO:7 or SEQ ID NO:8, or the corresponding full-length amino acid sequences. Preferably, the polypeptide contains at least 108, 93, 60 or 203 contiguous amino acids of SEQ ID NO:5, SEQ ID NO:6, or SEQ ID NO:7 or SEQ ID NO:8, respectively.

In preferred embodiments, the polypeptide comprises an amino acid sequence having (a) the amino acid sequence set forth in SEQ ID NO:5, SEQ ID NO:6, SEQ ID NO:7 or SEQ ID NO:8; (b) an amino acid sequence differing from that set forth in SEQ ID NO:5, SEQ ID NO:6, SEQ ID NO:7 or SEQ ID NO:8 by lacking one or more, but not all, of the following segments of amino acid residues: 1-1082, 1083-1257, 1258-1264, 1265-1372 or 1373-1519 of SEQ ID NO:5; 1-383 or 384-476 of SEQ ID NO:6; 1-227, 228-371 or 372-431 or SEQ ID NO:7 or 1-629 or 630-832 or SEQ ID NO:8; (c) the amino acid sequence set forth in SEQ ID NO:5, SEQ ID NO:6, SEQ ID NO:7 or SEQ ID NO:8 from amino acid residues 1-1082, 1083-1257, 1258-1264, 1265-1372 or 1373-1519 of SEQ ID NO:5; 1-383 or 384-476 of SEQ ID NO:6; 1-227, 228-371 or 372-431 or SEQ ID NO:7 or 1-629 or 630-832 or SEQ ID NO:8; or (d) an amino acid sequence differing from that set forth in SEQ ID NO:5, SEQ ID NO:6, SEQ ID NO:7 or SEQ ID NO:8 by lacking one or more, but not all, of the domains selected from the group consisting of a C-terminal domain, a PH domain, a DH domain, an N-terminal domain, an SH3 domain, a spacer region, a proline-rich region and a GTPase domain.

The polypeptide can be isolated from a natural source by methods well-known in the art. The natural source may be mammalian, preferably human, blood, semen or tissue, and the polypeptide may be synthesized using an automated polypeptide synthesizer. The isolated, enriched, or purified polypeptide is preferably: a Tks 107, Tks 113, Tks 118 or Tks 202 polypeptide.

In some embodiments the invention includes a recombinant polypeptide selected from the group consisting of Tks 107, Tks 113, Tks 118 and Tks 202. By "recombinant polypeptide" is meant a polypeptide produced by recombinant DNA techniques such that it is distinct from a naturally occurring polypeptide either in its location (e.g., present in a different cell or tissue than found in nature), purity or structure. Generally, such a recombinant polypeptide will be present in a cell in an amount different from that normally observed in nature.

In other aspects, the invention features an antibody (e.g., a monoclonal or polyclonal antibody) having specific binding affinity to a polypeptide or a polypeptide domain or fragment where the polypeptide is selected from the group consisting of Tks 107, Tks 113, Tks 118 and Tks 202. By "specific binding affinity" is meant that the antibody binds to the target polypeptide with greater affinity than it binds to other polypeptides under specified conditions. Antibodies or antibody fragments are polypeptides that contain regions that can bind other polypeptides. The term "specific binding affinity" describes an antibody that binds to a polypeptide with greater affinity than it binds to other polypeptides under specified conditions.

The term "polyclonal" refers to antibodies that are heterogenous populations of antibody molecules derived from the sera of animals immunized with an antigen or an antigenic functional derivative thereof. For the production of polyclonal antibodies, various host animals may be immunized by injection with the antigen. Various adjuvants may be used to increase the immunological response, depending on the host species.

"Monoclonal antibodies" are substantially homogenous populations of antibodies to a particular antigen. They may be obtained by any technique which provides for the production of antibody molecules by continuous cell lines in culture. Monoclonal antibodies may be obtained by methods known to those skilled in the art (Kohler *et al.,* Nature 256:495-497, 1975, and U.S. Patent No. 4,376,110, both of which are hereby incorporated by reference herein in their entirety including any figures, tables, or drawings).

The term "antibody fragment" refers to a portion of an antibody, often the hyper variable region and portions of the surrounding heavy and light chains, that displays specific binding affinity for a particular molecule. A hyper variable region is a portion of an antibody that physically binds to the polypeptide target.

Antibodies or antibody fragments having specific binding affinity to a polypeptide of the invention may be used in methods for detecting the presence and/or amount of polypeptide in a sample by probing the sample with the antibody under conditions suitable for polypeptide-antibody immunocomplex formation and detecting the presence and/or amount of the antibody conjugated to the polypeptide. Diagnostic kits for performing such methods may be constructed to include antibodies or antibody fragments specific for the polypeptide as well as a conjugate of a binding partner of the antibodies or the antibodies themselves.

An antibody or antibody fragment with specific binding affinity to a polypeptide of the invention can be isolated, enriched or purified from a prokaryotic or eukaryotic organism. Routine methods known to those skilled in the art enable production of antibodies or antibody fragments, in both prokaryotic and eukaryotic organisms. Purification, enrichment, and isolation of antibodies, which are polypeptide molecules, are described above.

Antibodies having specific binding affinity to a polypeptide of the invention may be used in methods for detecting the presence and/or amount of polypeptide in a sample by contacting the sample with the antibody under conditions such that an immunocomplex forms and detecting the presence and/or amount of the antibody conjugated to the polypeptide. Diagnostic kits for performing such methods may be constructed to include a first container containing the antibody and a second container having a conjugate of a binding partner of the antibody and a label, such as, for example, a radioisotope. The diagnostic kit may also include notification of an FDA approved use and instructions therefor.

Further aspects of the invention feature a hybridoma which produces an antibody having specific binding affinity to a polypeptide or a polypeptide domain, where the polypeptide is selected from the group consisting of Tks 107, Tks 113, Tks 118 and Tks 202. By "hybridoma" is meant an immortalized cell line that is capable of secreting an antibody, for example an antibody to a polypeptide of the invention. In preferred embodiments, the antibody to the polypeptide comprises a sequence of amino acids that is able to specifically bind a polypeptide of the invention.

In another aspect, the invention features a polypeptide binding agent able to bind to a polypeptide selected from the group consisting of Tks 107, Tks 113, Tks 118 and Tks 202. The binding agent is preferably a purified antibody that recognizes an epitope present on a polypeptide of the invention. Other binding agents include molecules that bind to polypeptides and analogous molecules that bind to a polypeptide. Such binding agents may be identified by using assays that measure binding partner activity.

The invention also features a method for screening for human cells containing a polypeptide of the invention or an equivalent sequence. The method involves identifying the novel polypeptide in human cells using techniques that are routine and standard in the art, such as those described herein for identifying the polypeptides of the invention (e.g., cloning, Southern or Northern blot analysis, in situ hybridization, PCR amplification, etc.).

In yet another aspect, the invention features methods for identifying a substance that modulates polypeptide activity comprising the steps of: (a) contacting a polypeptide selected from the group consisting of Tks 107, Tks 113, Tks 118 and Tks 202 with a test substance; (b) measuring the activity of said polypeptide; and (c) determining whether said substance modulates the activity of said polypeptide.

The term "modulates" refers to the ability of a compound to alter the function of a polypeptide of the invention. A modulator preferably activates or inhibits the activity of a polypeptide of the invention depending on the concentration of the compound exposed to the polypeptide.

The term "activates" refers to increasing the cellular activity of the polypeptide. The term "inhibit" refers to decreasing the cellular activity of the peptide. Activity preferably affects the interaction with a natural binding partner, such as src.

The term "modulates" also refers to altering the function of polypeptides of the invention by increasing or decreasing the probability that a complex forms between the polypeptide and a natural binding partner. A modulator preferably increases the probability that such a complex forms between the polypeptide and the natural binding partner, more preferably increases or decreases the probability that a complex forms depending on the concentration of the compound exposed, and most preferably decreases the probability that a complex forms.

The term "complex" refers to an assembly of at least two molecules bound to one another. Signal transduction complexes often contain at least two protein molecules bound to one another.

The term "natural binding partner" refers to polypeptides, lipids, small molecules or nucleic acids that bind to polypeptides in cells. A change in the interaction between a polypeptide and a natural binding partner can manifest itself as an increased or decreased probability that the interaction forms or an increased or decreased concentration of polypeptide/natural binding partner complex.

The term "contacting" as used herein refers to mixing a solution comprising the test compound with a liquid medium bathing the cells of the methods. The solution comprising the compound may also comprise another component, such as dimethyl sulfoxide (DMSO), which facilitates the uptake of the test compound or compounds into the cells of the methods. The solution comprising the test compound may be added to the medium bathing the cells by utilizing a delivery apparatus, such as a pipet-based device or syringe-based device.

Other aspects of the invention feature methods for identifying a substance that modulates polypeptide activity in a cell comprising the steps of: (a) expressing a polypeptide in a cell, wherein said polypeptide is selected from the group consisting of Tks 107, Tks 113, Tks 118 and Tks 202; (b) adding a test substance to said cell; and (c) monitoring a change in cell phenotype or the interaction between said polypeptide and a natural binding partner.

The term "expressing" as used herein refers to the production of polypeptide of the invention from a nucleic acid vector within a cell. The nucleic acid vector is transfected into cells using well known techniques in the art as described herein.

Another aspect of the invention provides methods for treating a disease by administering to a patient in need of such treatment a substance that modulates the activity of a polypeptide selected from the group consisting of Tks 107, Tks 113, Tks 118 and Tks 202. Preferably, the disease is selected from the group consisting of rheumatoid arthritis, atherosclerosis, autoimmune disorders, organ transplantation, myocardial infarction, cardiomyopathies, stroke, renal failure, oxidative stress-related neurodegenerative disorders and cancer.

Substances useful for treatment of disorders or diseases preferably show positive results in one or more *in vitro* assays for an activity corresponding to treatment of the disease or disorder in question. Substances that modulate the activity of the polypeptides preferably include, but are not limited to, antisense oligonucleotides and inhibitors of protein kinases.

The term "preventing" refers to decreasing the probability that an organism contracts or develops an abnormal condition.

The term "treating" refers to having a therapeutic effect and at least partially alleviating or abrogating an abnormal condition in the organism.

The term "therapeutic effect" refers to the inhibition or activation factors causing or contributing to the abnormal condition. A therapeutic effect relieves to some extent one or more of the symptoms of the abnormal condition. In reference to the treatment of abnormal conditions, a therapeutic effect can refer to one or more of the following: (a) an increase or decrease in the proliferation, growth, and/or differentiation of cells; (b) inhibition (i.e., slowing or stopping) or acceleration of cell death; (c) inhibition or acceleration of degeneration; (d) relieving to some extent one or more of the symptoms associated with the abnormal condition; and (e) enhancing the function of the affected population of cells. Compounds demonstrating efficacy against abnormal conditions can be identified as described herein.

The term "abnormal condition" refers to a function in the cells or tissues of an organism that deviates from their normal functions in that organism. An abnormal condition can relate to cell proliferation, cell differentiation or cell survival.

Abnormal cell proliferative conditions include cancers such as fibrotic and mesangial disorders, abnormal angiogenesis and vasculogenesis, wound healing, psoriasis, diabetes mellitus and inflammation.

Abnormal differentiation conditions include, but are not limited to, neurodegenerative disorders, slow wound healing rates and slow tissue grafting healing rates.

Abnormal cell survival conditions relate to conditions in which programmed cell death (apoptosis) pathways are activated or abrogated. A number of protein kinases are associated with the apoptosis pathways. Aberrations in the function of any one of the protein kinases could lead to cell immortality or premature cell death.

The term "aberration", in conjunction with the function of a kinase in a signal transduction process, refers to a kinase that is over- or under-expressed in an organism, mutated such that its catalytic activity is lower or higher than wild-type protein kinase activity, mutated such that it can no longer interact with a natural binding partner, is no longer modified by another protein kinase or protein phosphatase, or no longer interacts with a natural binding partner.

The term "administering" relates to a method of incorporating a compound into cells or tissues of an organism. The abnormal condition can be prevented or treated when the cells or tissues of the organism exist within the organism or outside of the organism. Cells existing outside the organism can be maintained or grown in cell culture dishes. For cells harbored within the organism, many techniques exist in the art to administer compounds, including (but not limited to) oral, parenteral, dermal, injection, and aerosol applications. For cells outside of the organism, multiple techniques exist in the art to administer the compounds, including (but not limited to) cell microinjection techniques, transformation techniques and carrier techniques.

The abnormal condition can also be prevented or treated by administering a compound to a group of cells having an aberration in a signal transduction pathway to an organism. The effect of administering a compound on organism function can then be monitored. The organism is preferably a mouse, rat, rabbit, guinea pig or goat, more preferably a monkey or ape, and most preferably a human.

Another aspect of the invention features methods for detection of a polypeptide (or nucleic acid that encodes a polypeptide) in a sample as a diagnostic tool for diseases or disorders, wherein the method comprises the steps of: (a) contacting the sample with a nucleic acid probe which hybridizes under hybridization assay conditions to a nucleic acid target region of a polypeptide selected from the group consisting of Tks 107, Tks 113, Tks 118 and Tks 202, said probe comprising the nucleic acid sequence encoding the polypeptide, fragments thereof, and the complements of the sequences and fragments; and (b) detecting the presence or amount of the probe:target region hybrid as an indication of the disease.

In preferred embodiments of the invention, the diseases or disorders are selected from the group consisting of rheumatoid arthritis, atherosclerosis, autoimmune disorders, organ transplantation, myocardial infarction, cardiomyopathies, stroke, renal failure, oxidative stress-related neurodegenerative disorders and cancer.

Hybridization conditions should be such that hybridization occurs only with the genes in the presence of other nucleic acid molecules. Under stringent hybridization conditions only highly complementary nucleic acid sequences hybridize. Preferably, such conditions prevent hybridization of nucleic acids having 1 or 2 mismatches out of 20 contiguous nucleotides. Such conditions are defined supra.

The diseases for which detection of genes in a sample could be diagnostic include diseases in which nucleic acid (DNA and/or RNA) is amplified in comparison to normal cells. By "amplification" is meant increased numbers of DNA or RNA in a cell compared with normal cells. "Amplification" as it refers to RNA can be an increase in nucleic acid to indicate a detectable presence of RNA in cells, since in some normal cells there is no basal expression of RNA. In other normal cells, a basal level of expression exists, therefore in these cases amplification is an increase in nucleic acid to indicate an increase in detectable presence of at least 1-2-fold, and preferably more, compared to the basal level.

Diseases that could be diagnosed by detection of nucleic acid in a sample preferably include cancers. The test samples suitable for nucleic acid probing methods of the present invention include, for example, cells or nucleic acid extracts of cells, or biological fluids. The samples used in the above-described methods will vary based on the assay format, the detection method and the nature of the tissues, cells or extracts to be assayed. Methods for preparing nucleic acid extracts of cells are well known in the art and can be readily adapted in order to obtain a sample that is compatible with the method utilized.

Finally, the invention is drawn to transgenic animals and gene therapy using tks 107, tks 113, tks 118 and tks 202.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

The summary of the invention described above is not limiting and other features and advantages of the invention will be apparent from the following detailed description of the invention, and from the claims.

### BRIEF DESCRIPTION OF THE FIGURES

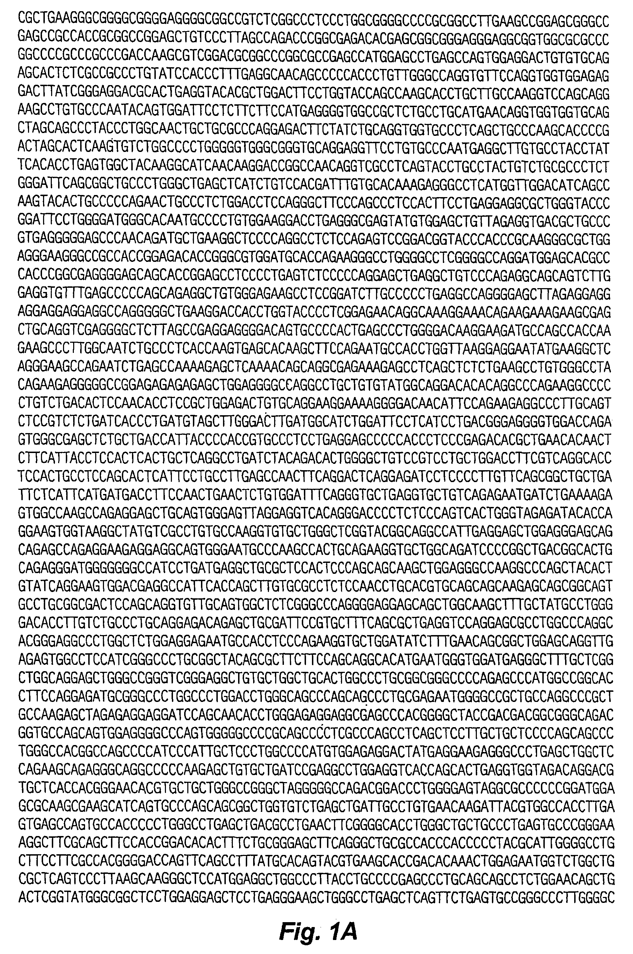

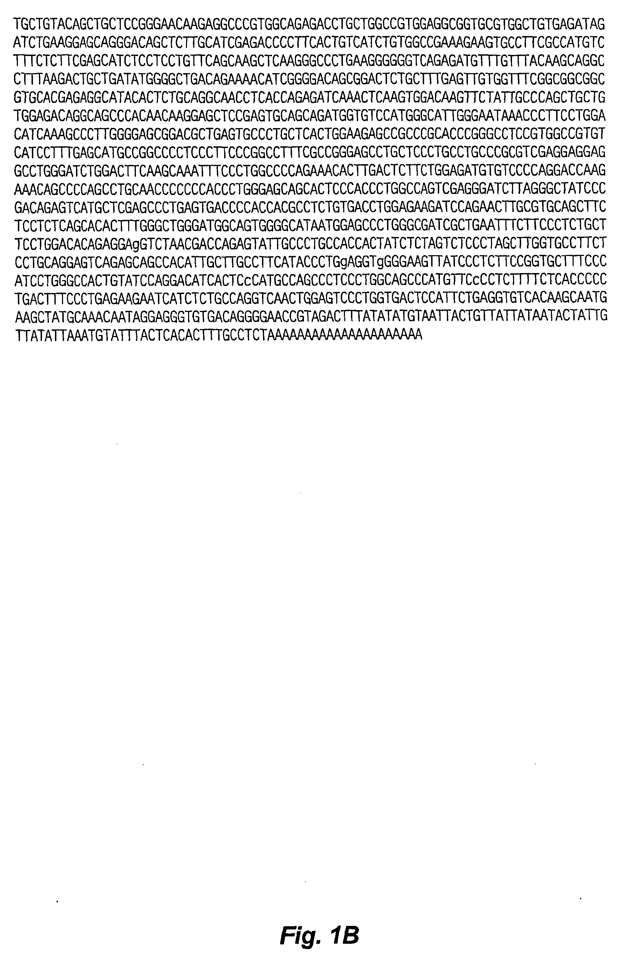

Figure 1 shows the nucleotide sequence for tks 107 (SEQ ID NO:1);

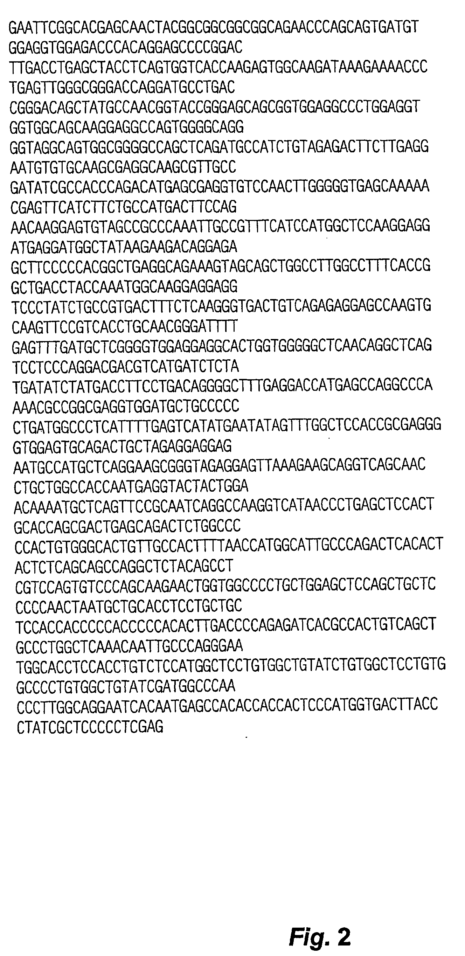

Figure 2 shows the nucleotide sequence for tks 113 (SEQ.ID NO:2);

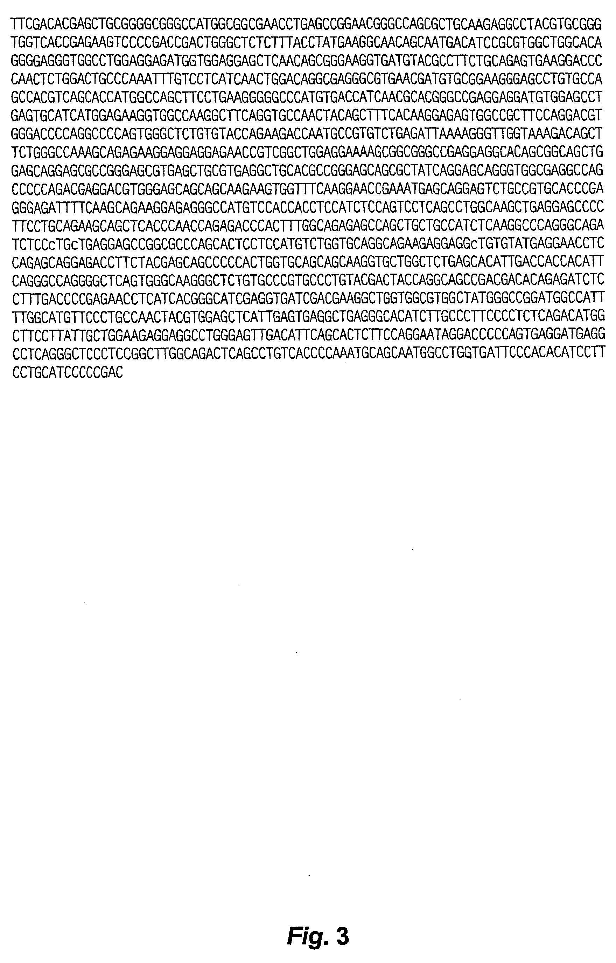

Figure 3 shows the nucleotide sequence for tks 118 (SEQ ID NO:3);

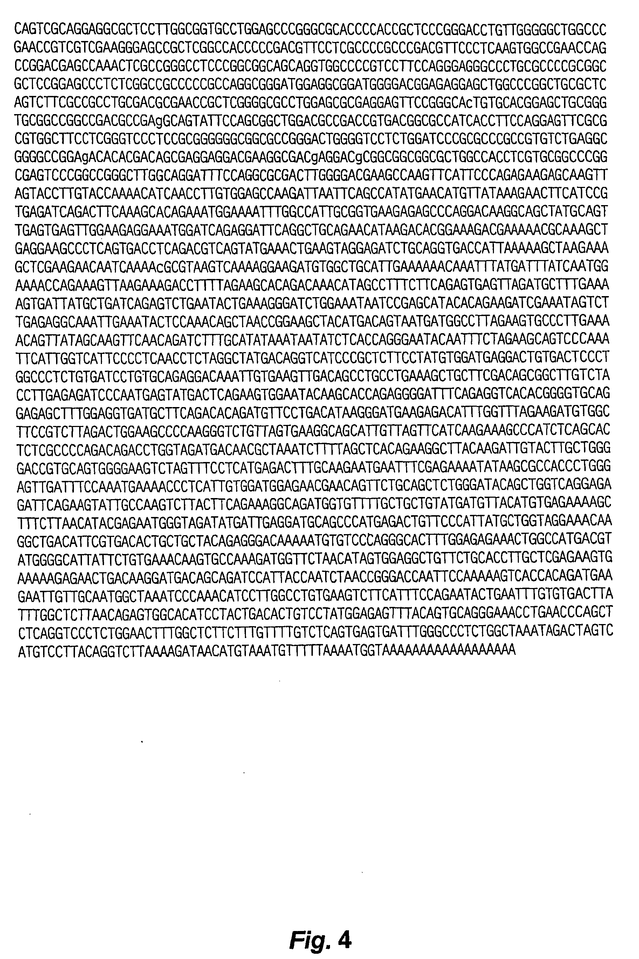

Figure 4 shows the nucleotide sequence for tks 202 (SEQ ID NO:4);

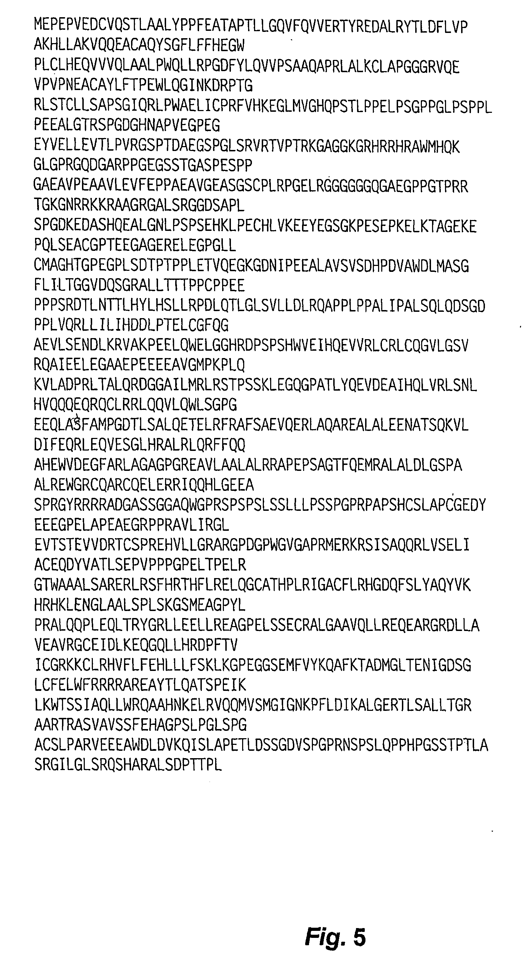

Figure 5 shows the purported translated amino acid sequence for tks 107 (SEQ ID NO:5);

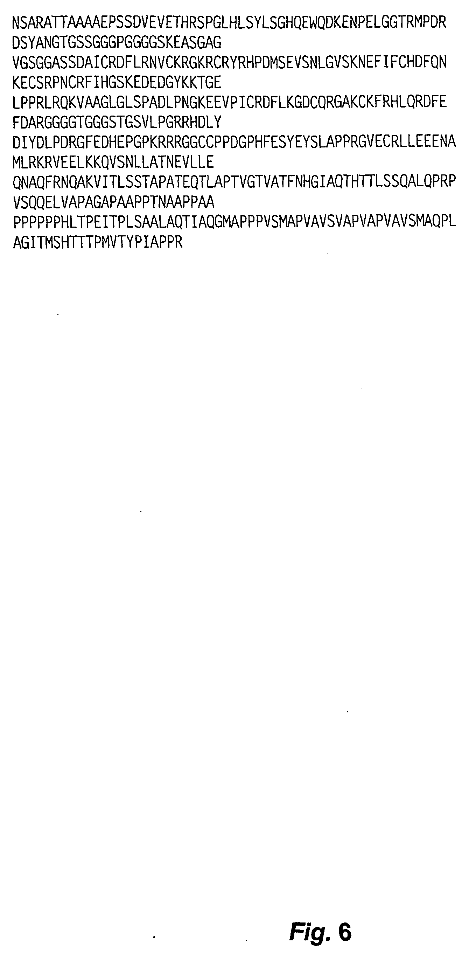

Figure 6 shows the purported translated amino acid sequence for tks 113 (SEQ ID NO:6);

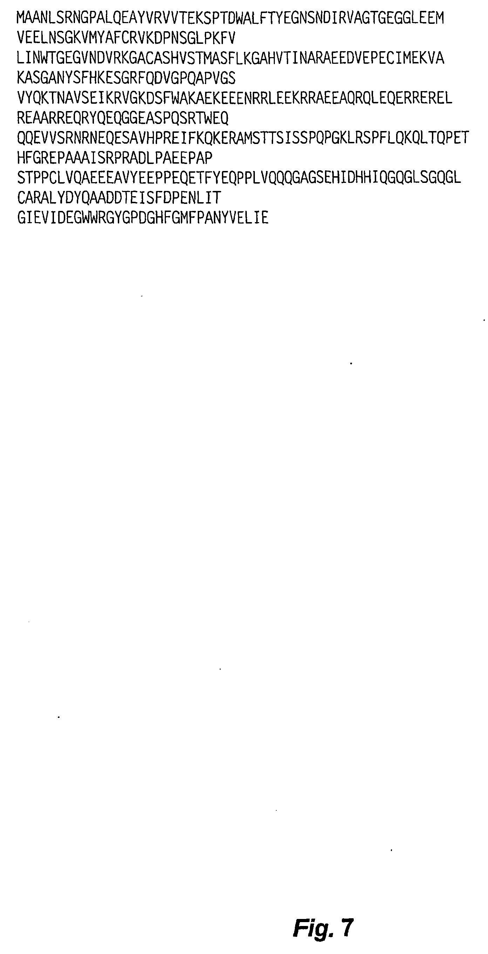

Figure 7 shows the purported translated amino acid sequence for tks 118 (SEQ ID NO:7);

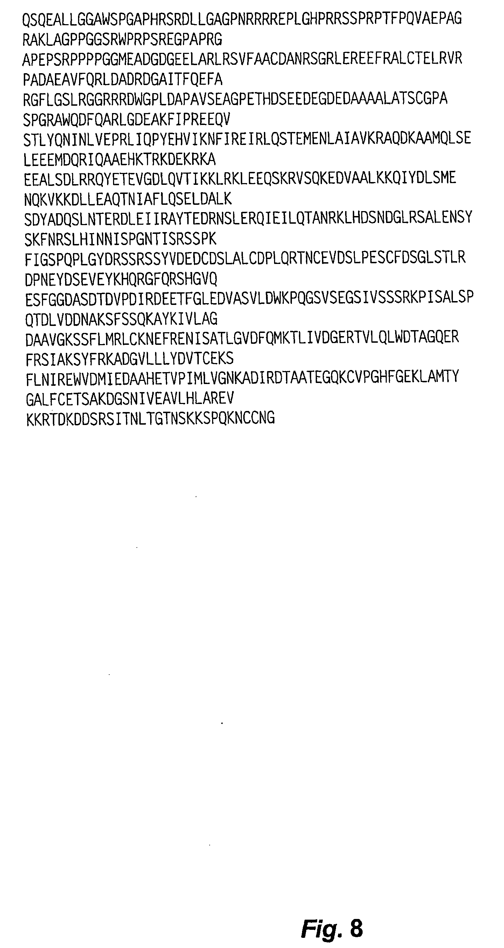

Figure 8 shows the purported translated amino acid sequence for tks 202 (SEQ ID NO:8);

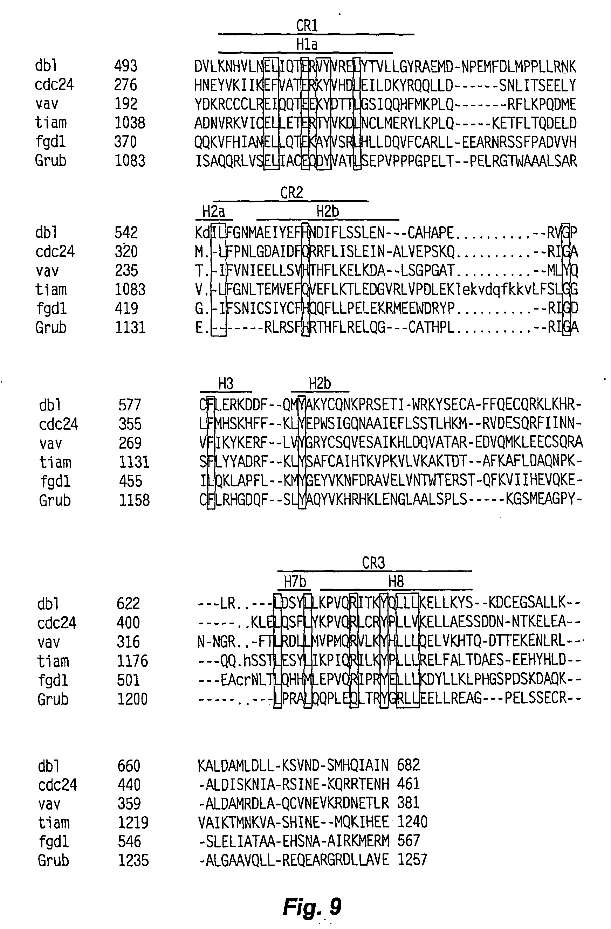

Figure 9 shows a Hidden Markov Model (HMM) alignment of the dbl homology (DH) domains of dbl, cdc24, vav, tiam, fgdl and Grub; the bars above the sequence represent important α-helices as predicted from crystal structure of the DH/PH domain of h-sos (H1-H8) or conserved regions (CR1-CR3); the few well conserved amino acids are shaded (Soisson, S., *et al.* (1988) Cell 95:259-268);

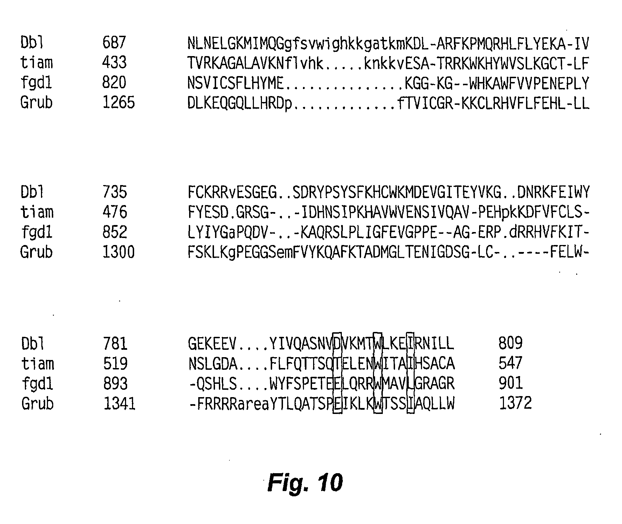

Figure 10 shows a Hidden Markov Model (HMM) alignment of the Grub Pleckstrin Homology (PH) domain with the PH domains of dbl, tiam, and fgdl; the shaded amino acids represent the few well conserved amino acids found in PH domains (Shaw, G. (1996) BioEssays 18: 35-46);
Figure 11 shows expression of Grub in various tissues, where the original Tks 107 clone was used to probe Multiple Tissue Northern Blots;
Figure 12 shows Grub expression in normal tissues and NCI tumor cells, where the original Tks 107 clone was use to probe total RNA from various sources; the top 2 panels contain RNA from normal tissues and the remaining panels contain RNA from various tumor cell lines;

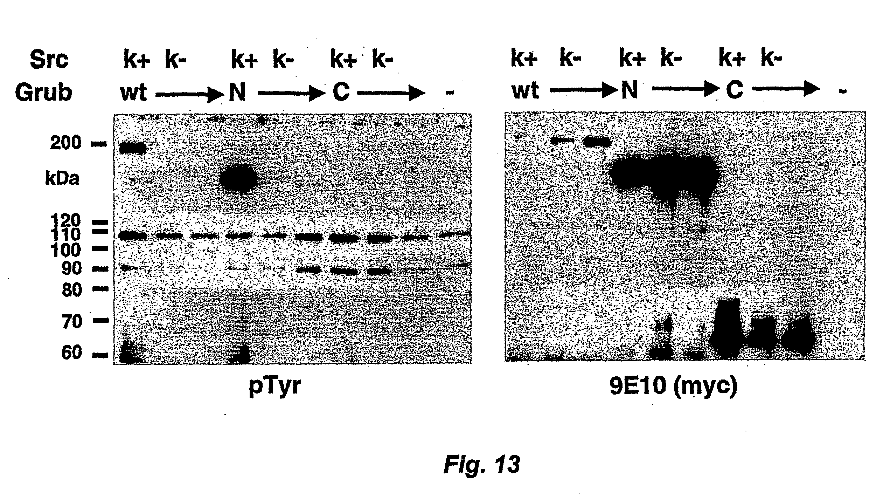

Figure 13 shows coexpression of Grub with Src in 293 cells. Constructs expressing myc tagged, full length (wt), N-terminal (N), or C-terminal (C); of Grub were co-expressed in 293 cells with activated Src (k+), kinase dead Src (k-), or no Src; lysates were immunoprecipitated with α-myc antibodies (9E10) and western blotted with 9E10 or with α-phosphotyrosine antibodies (pTyr); only the full length or N terminal Grub is phosphorylated by activated Src; no phosphorylation is observed when C terminal Grub is co-expressed with activated Src or when any Grub constructs are co-expressed with kinase dead Src;
Figure 14 shows expression of Tks113 in various tissues; a 1.5 kb EcoRI fragment from Tks113 was used to probe Multiple Tissue Northerns (Clontech);
Figure 15 shows Tks 113 specific antibodies. Lanes 1 and 3 contain cell lysates from NIH3T3 cells and lanes 2 and 4 contain cell lysates from NCI H460 cells; panel A was immunoblotted with rabbit preimmune serum; panel B was immunoblotted with serum from a rabbit immnunized with Gst-Tks113;

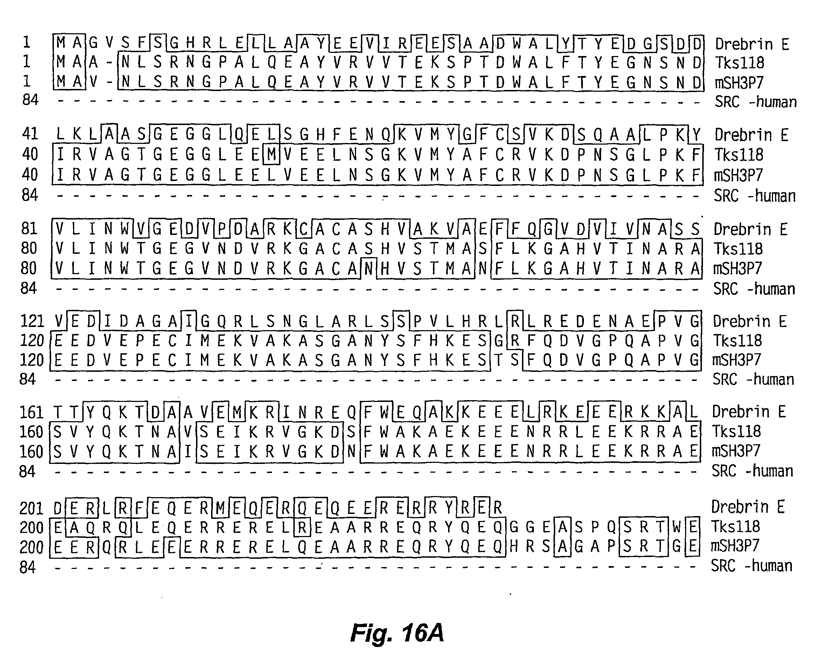

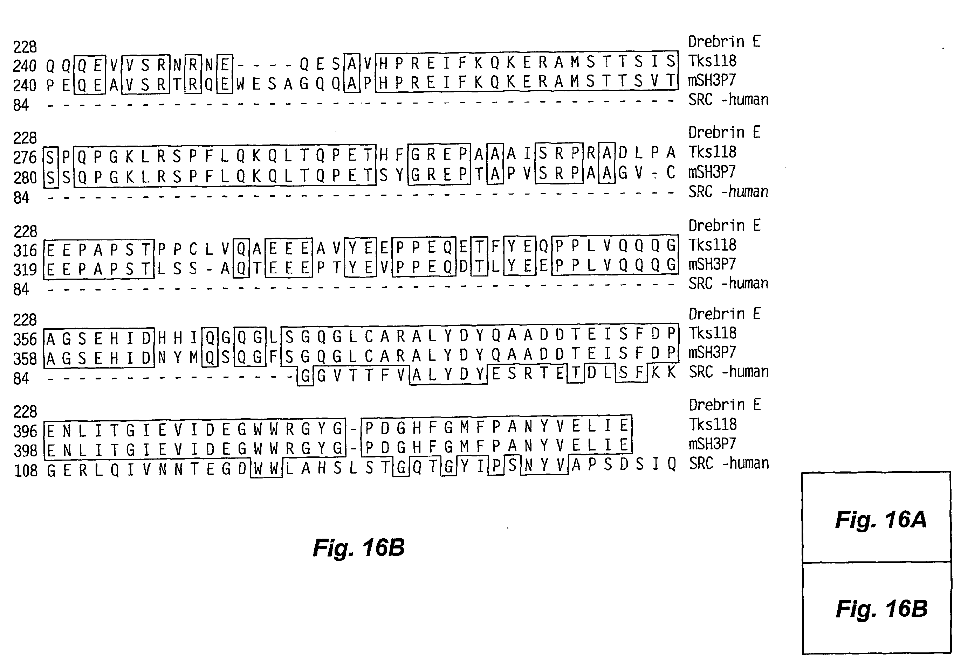

Figure 16 shows amino acid sequence alignment (MegAlign using the J. Hein method) of Tks 118 with mSH3P7, Drebrin E and the SH3 domain of Src;
Figure 17 shows expression of Tks118 in various tissues, where a 1.6 kb EcoRI/XhoI fragment from Tks118 was used to probe Multiple Tissue Northerns (Clontech);
Figure 18 shows Tks118 expression in normal tissues and NCI tumor cells; the original Tks118 clone was used to probe total RNA from various sources; the top 2 panels contain RNA from normal tissues; the remaining panels contain RNA from various tumor cell lines;
Figure 19 shows coexpression of Tks118 with Src in 293 cells; constructs expressing myc tagged Tks119 was co-expressed in 293 cells with activated Src (k+), kinase dead Src (k-) or empty vector control (-); lysates were immunoprecipitated with a -myc antibodies (9E10) and western blotted with 9E10 or with a-phosphotyrosine antibodies; note that Tks118 is only phosphorylated when it is coexpressed with activated (but not kinase dead) Src;
Figure 20 shows co-staining of NIH3T3-Y527F cells with α-Tks118 (α-5033) and α-Vinculin (Upstate Biotechnology); the stain shows that Tks118 staining co-localizes with Vinculin, which stains the round podosomal structures at the ends of cell projections seen in these cells;

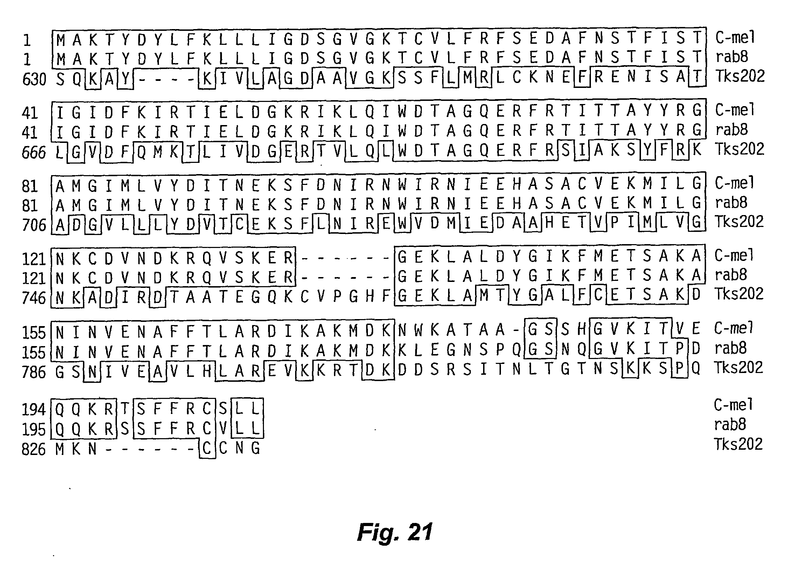

Figure 21 shows Alignment of the C-terminal of Tks 202 with c-mel and rab8 (MegAlign using Clustal method);
Figure 22 shows Expression of Tks202 in various tissues; the 5' 500 b.p. of the original Tks202 clone was used to probe Multiple Tissue Northerns (Clontech);

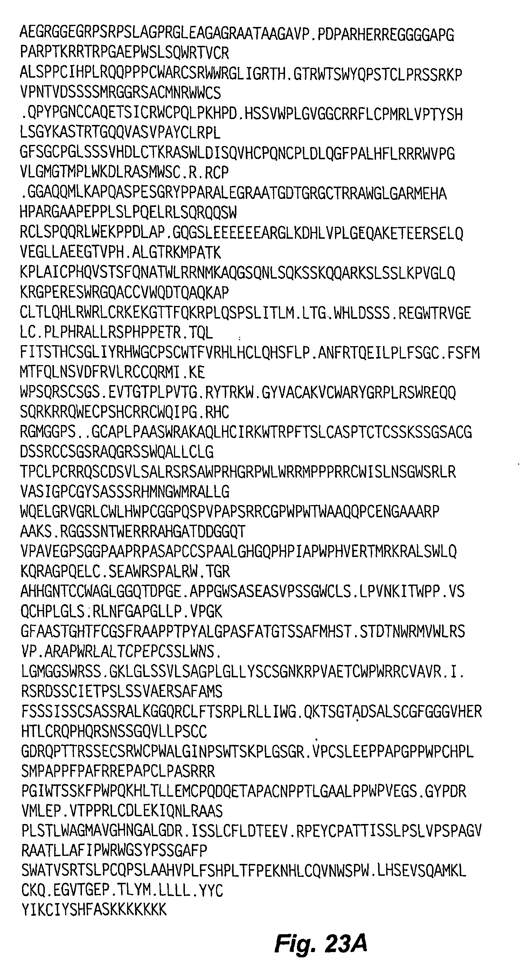

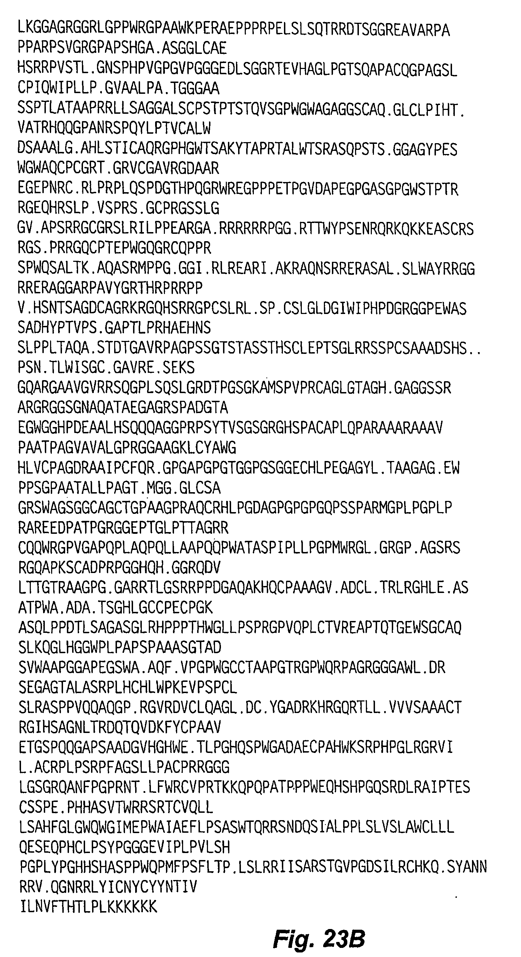

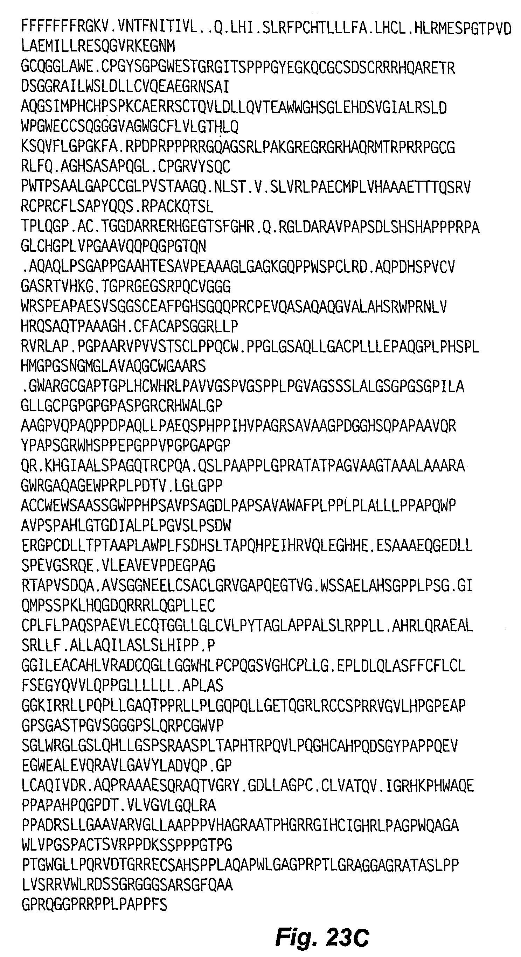

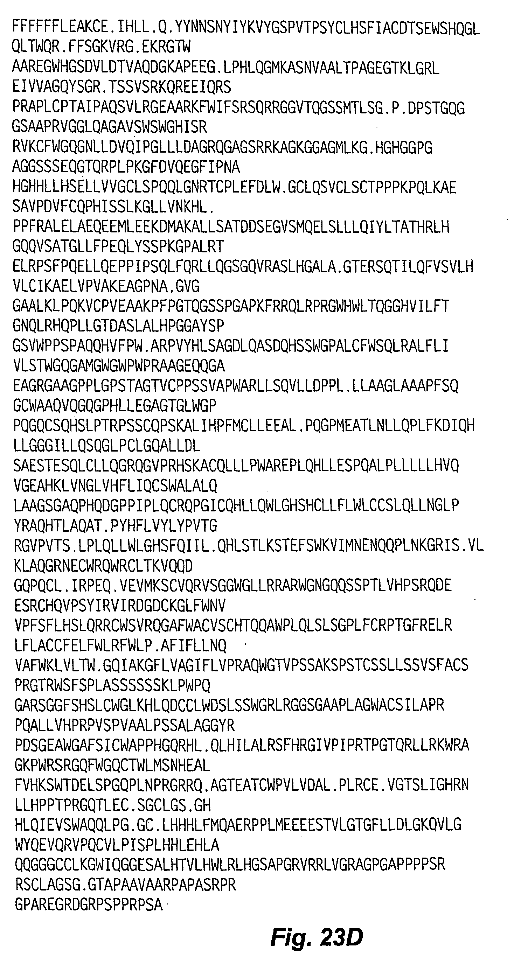

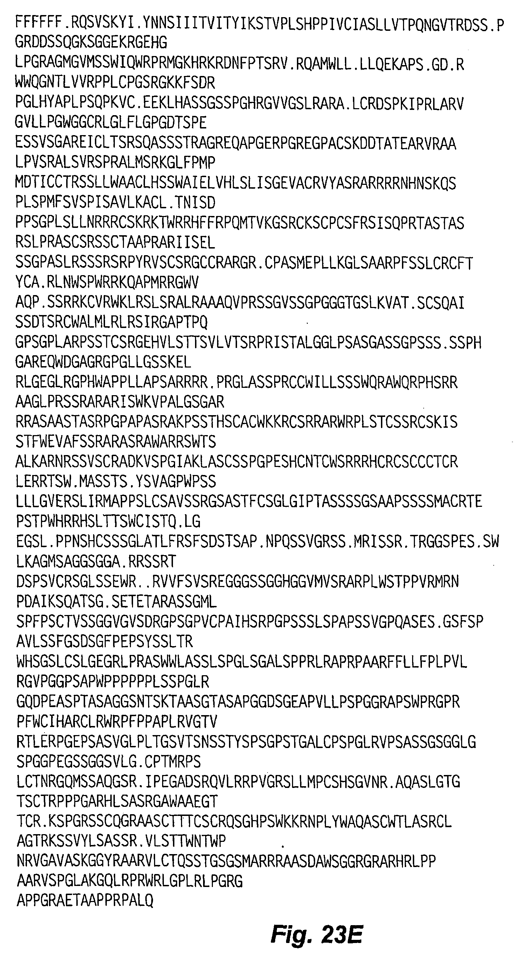

Figure 23 shows translations in 5 other reading frames for Grub;

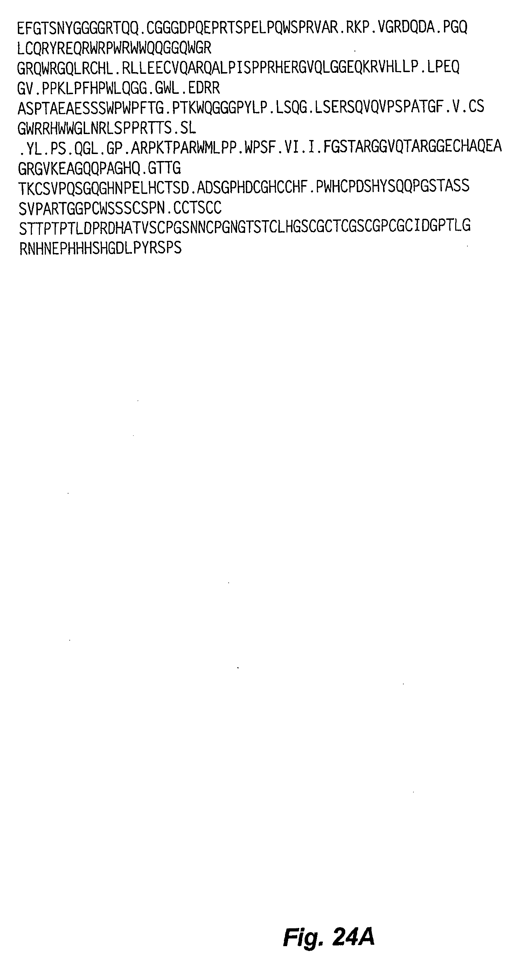

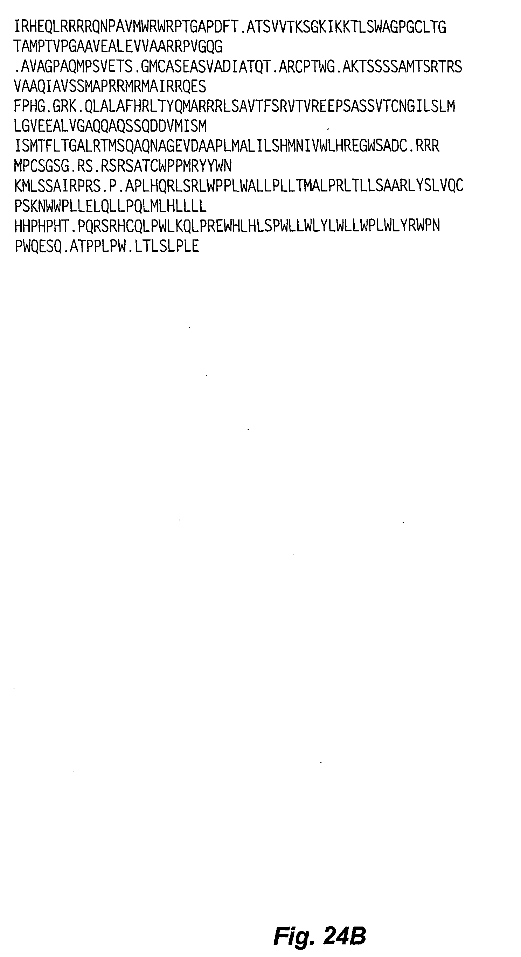

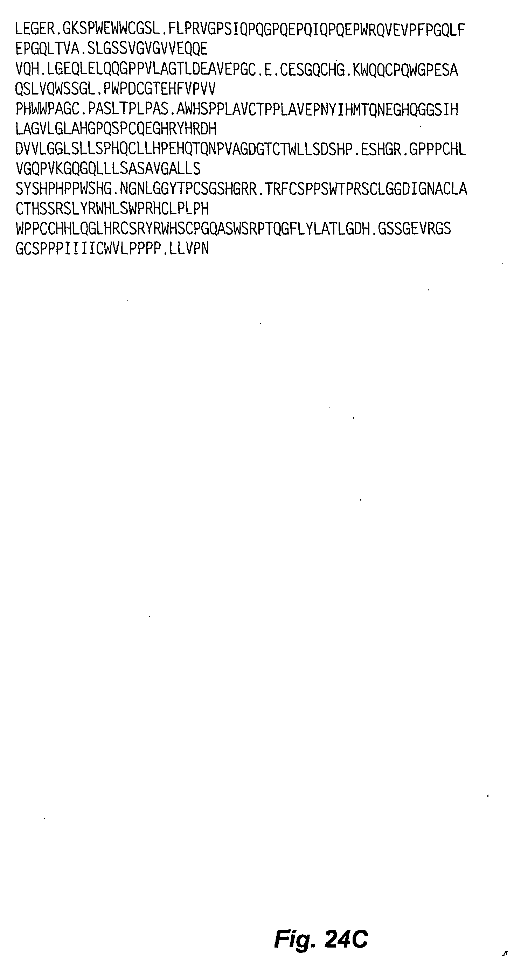

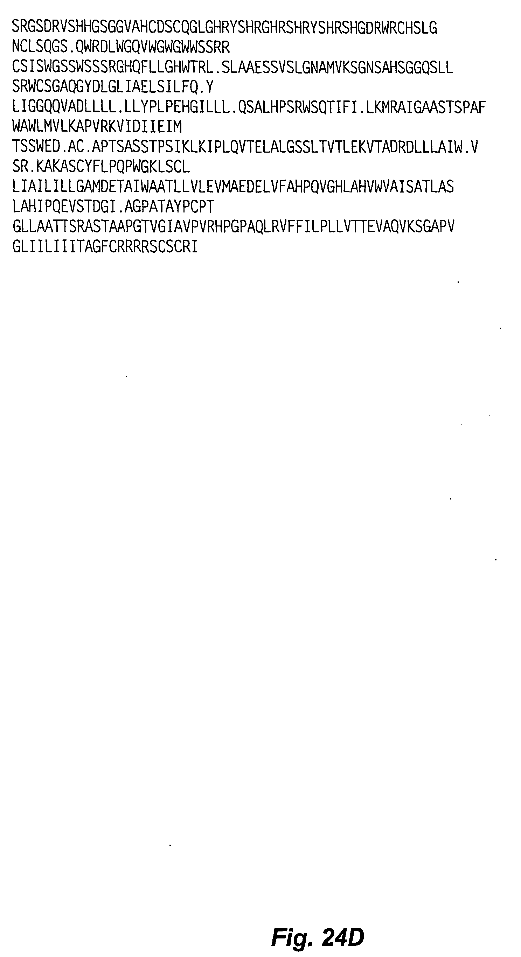

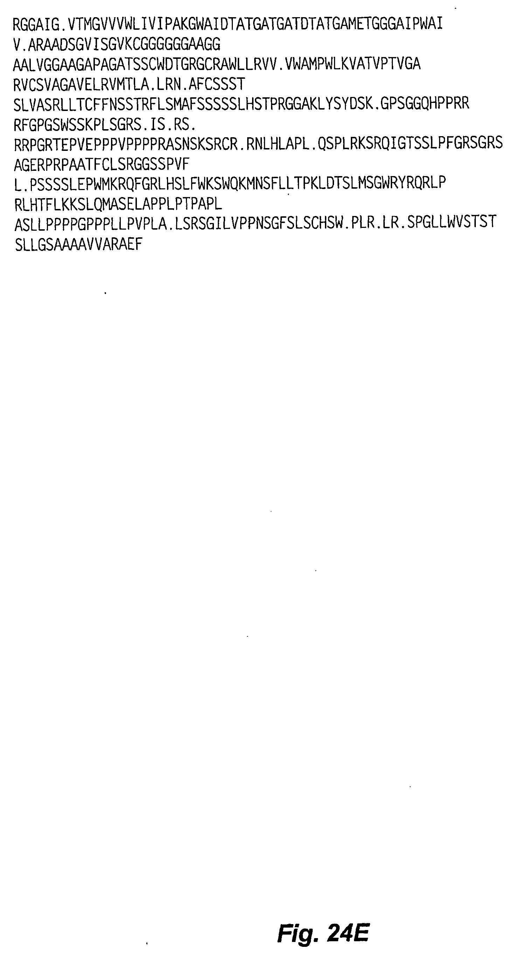

Figure 24 shows translation in 5 other reading frames for Tks 113;

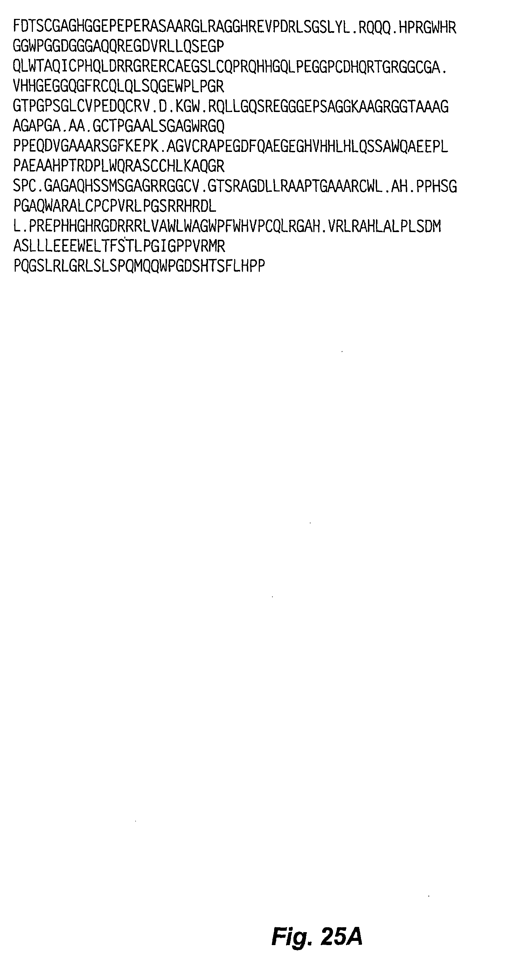

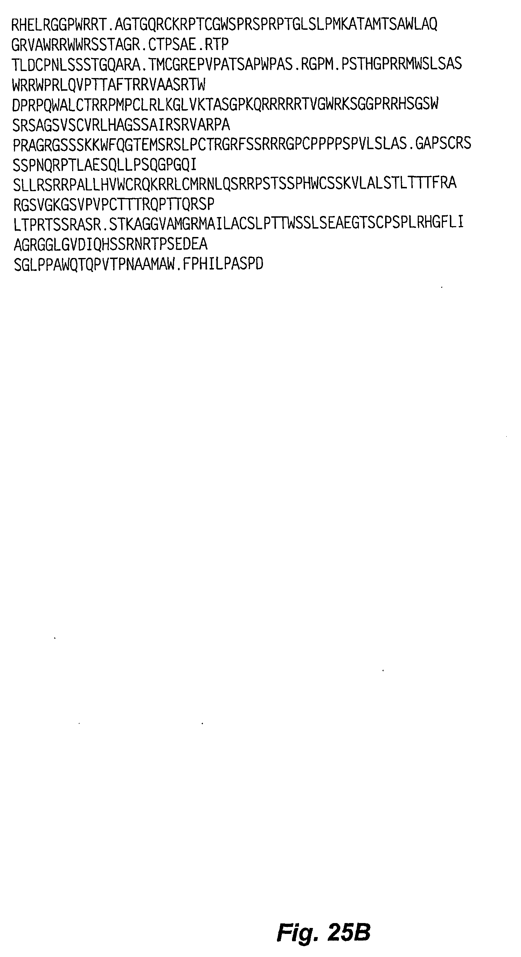

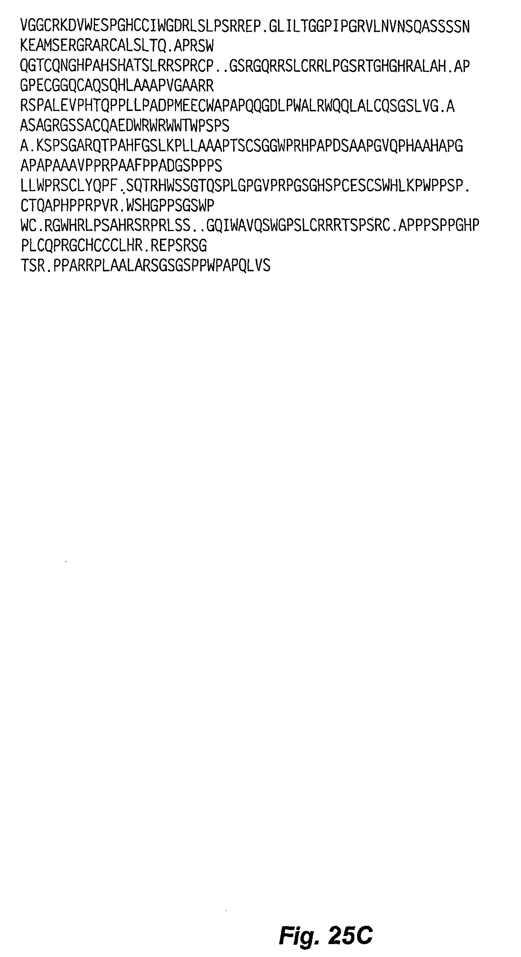

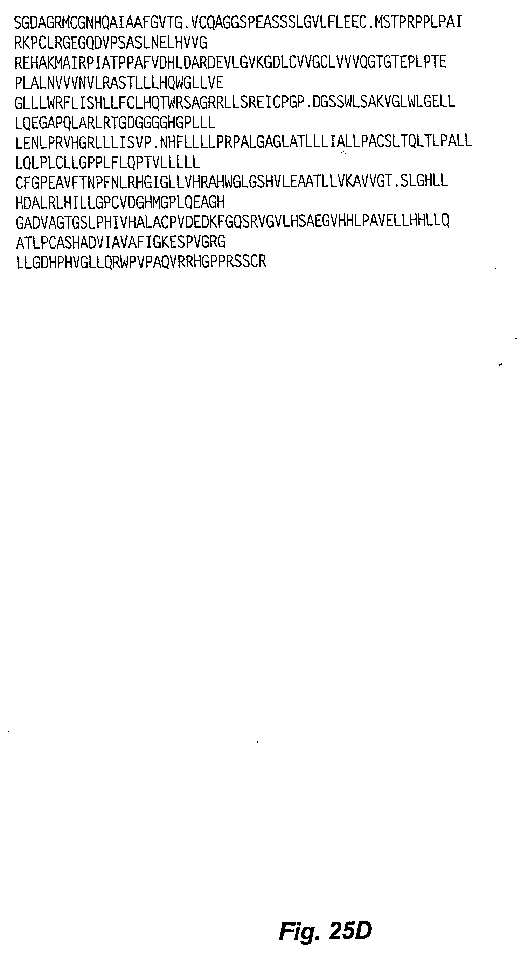

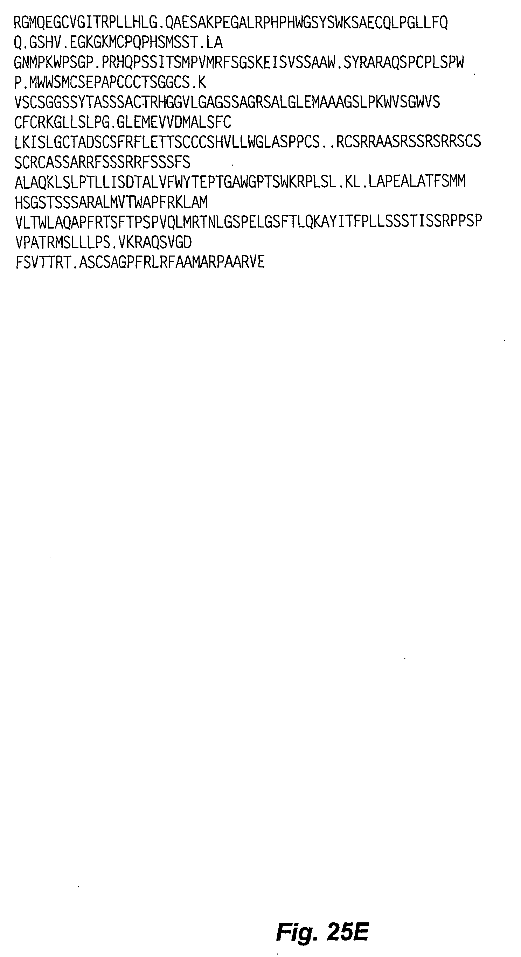

Figure 25 shows translation in 5 other reading frames for Tks 118; and

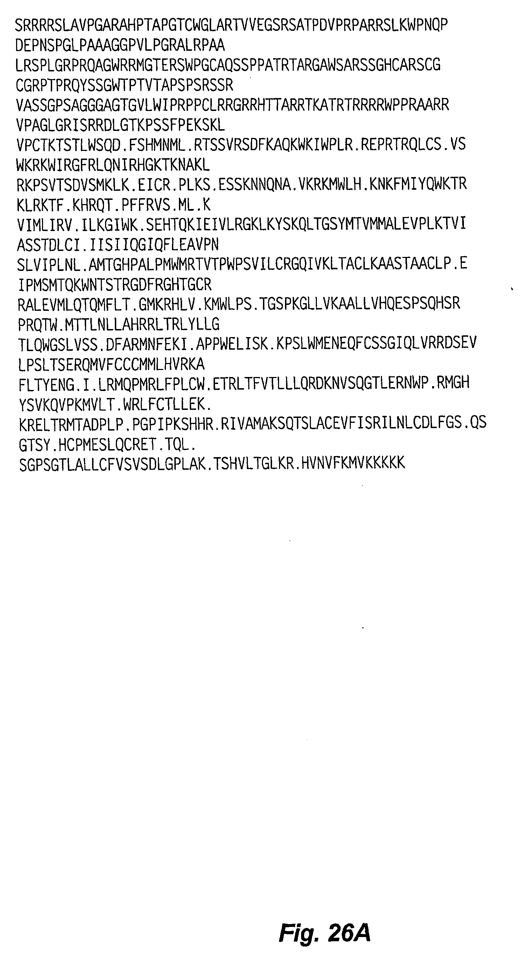

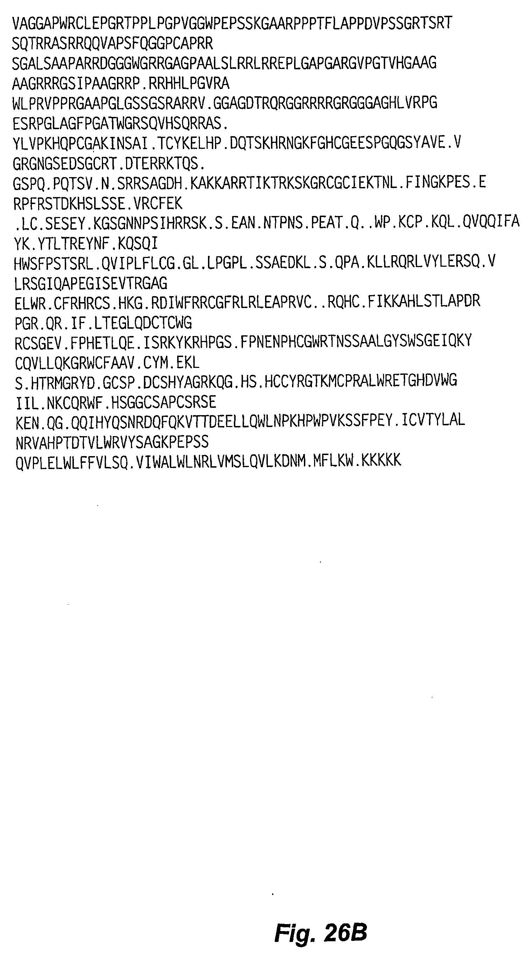

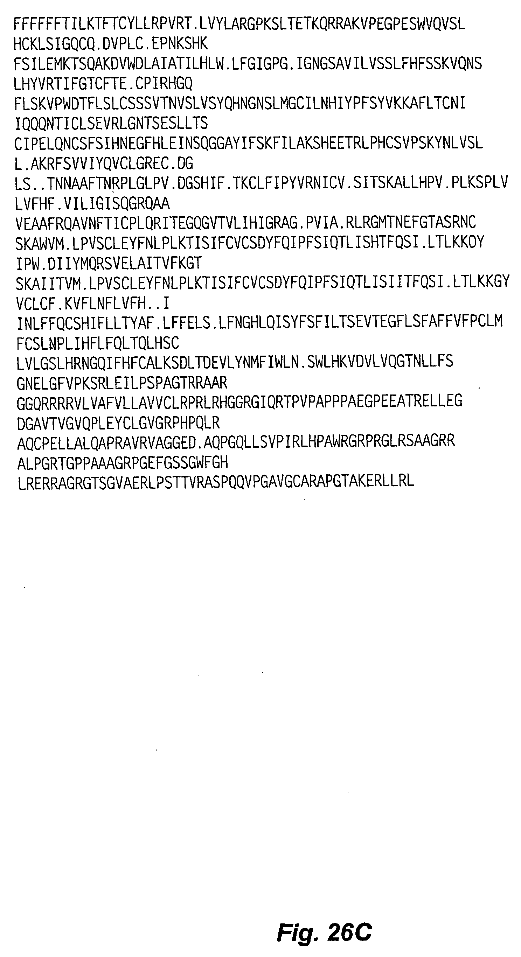

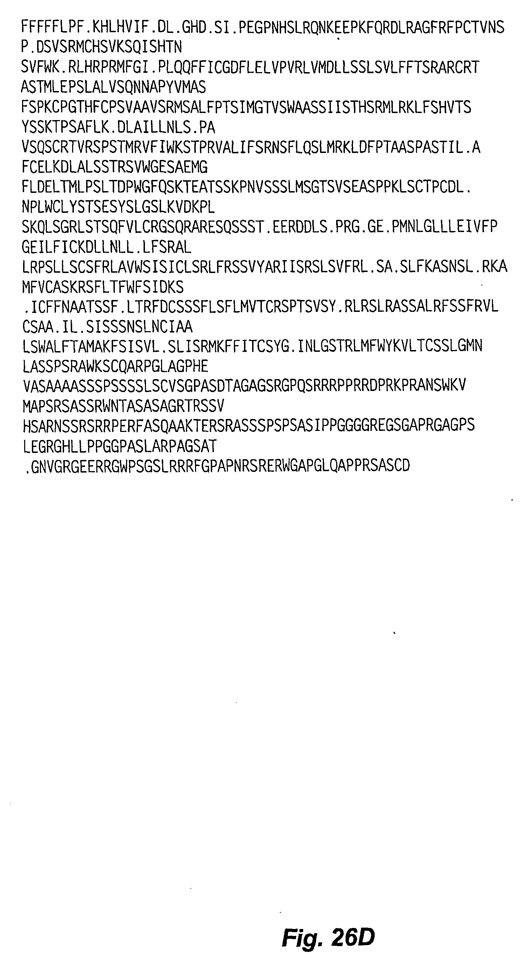

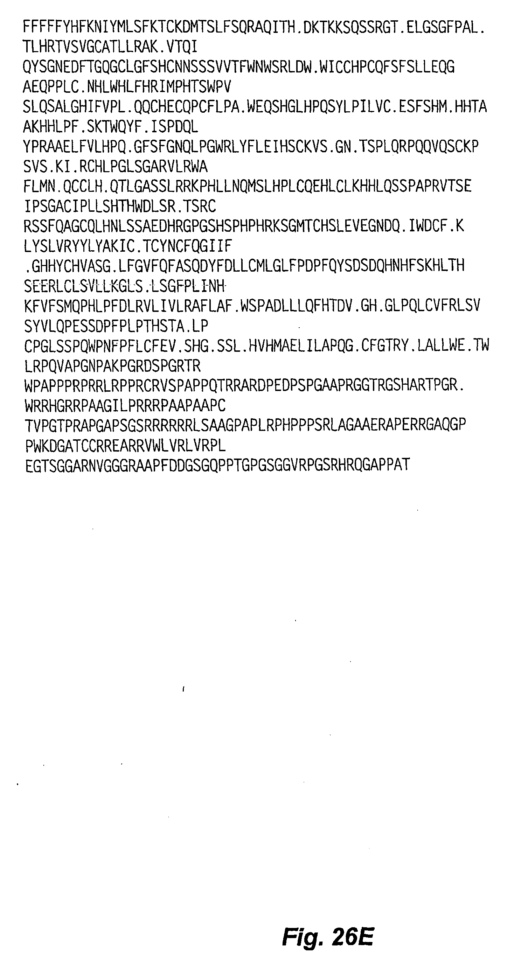

Figure 26 shows translation in 5 other reading frames for Tks 202.

### DETAILED DESCRIPTION OF THE INVENTION

The present invention relates in part to the polypeptides, nucleic acids encoding such polypeptides, cells containing such nucleic acids, antibodies to such polypeptides, assays utilizing such polypeptides, and methods relating to all of the foregoing with respect to the novel substrates of the invention, Tks 107, Tks 113, Tks 118 and Tks 202. The polypeptides and nucleic acids may be produced using well-known and standard synthesis techniques when given the sequences presented herein. The examples below demonstrate the isolation and characterization of the proteins tks 107/Grub, tks 113, tks 118/Dresh and tks 202 (tyrosine kinase substrate).

Protein tyrosine kinases comprise a large superfamily of related proteins that function to transduce signals from the extracellular environment to the interior of the cell. These signals direct all aspects of cellular functions, including growth, differentiation, and death. For any particular kinase, identification of the relevant substrates is an important step in elucidation of its function.

We set out to identify novel substrates of the cytoplasmic tyrosine kinase Src. We employed a screening strategy based on the in vitro phosphorylation of lambda expression libraries immobilized on nitrocellulose filters by Src containing Sf9 lysates (Lock, P. et. al. EMBO J. 17(15):4346-4357, 1998). Lambda clones encoding 4 Src substrates were identified in this manner, tks 107, 113, 118 and tks 202.

### I. The Nucleic Acids of the Invention

Included within the scope of this invention are the functional equivalents of the herein-described isolated nucleic acid molecules. The degeneracy of the genetic code permits substitution of certain codons by other codons that specify the same amino acid and hence would give rise to the same protein. The nucleic acid sequence can vary substantially since, with the exception of methionine and tryptophan, the known amino acids can be coded for by more than one codon. Thus, portions or all of the genes of the invention could be synthesized to give a nucleic acid sequence significantly different from that shown in SEQ ID NO:1, SEQ ID NO:2, SEQ ID NO:3 and SEQ ID NO:4. The encoded amino acid sequence thereof would, however, be preserved.

In addition, the nucleic acid sequence may comprise a nucleotide sequence which results from the addition, deletion or substitution of at least one nucleotide to the 5'-end and/or the 3'-end of the nucleic acid formula shown in SEQ ID NO:1, SEQ ID NO:2, SEQ ID NO:3 or SEQ ID NO:4, or a derivative thereof. Any nucleotide or polynucleotide may be used in this regard, provided that its addition, deletion or substitution does not alter the amino acid sequence of SEQ ID NO:5, SEQ ID NO:6, SEQ ID NO:7 or SEQ ID NO:8, which is encoded by the nucleotide sequence. For example, the present invention is intended to include any nucleic acid sequence resulting from the addition of ATG as an initiation codon at the 5'-end of the inventive nucleic acid sequence or its derivative, or from the addition of TTA, TAG or TGA as a termination codon at the 3'-end of the inventive nucleotide sequence or its derivative. Moreover, the nucleic acid molecule of the present invention may, as necessary, have restriction endonuclease recognition sites added to its 5'-end and/or 3'-end.

Such functional alterations of a given nucleic acid sequence afford an opportunity to promote secretion and/or processing of heterologous proteins encoded by foreign nucleic acid sequences fused thereto. All variations of the nucleotide sequence of the genes of the invention and fragments thereof permitted by the genetic code are, therefore, included in this invention.

Further, it is possible to delete codons or to substitute one or more codons with codons other than degenerate codons to produce a structurally modified polypeptide, but one which has substantially the same utility or activity as the polypeptide produced by the unmodified nucleic acid molecule. As recognized in the art, the two polypeptides are functionally equivalent, as are the two nucleic acid molecules that give rise to their production, even though the differences between the nucleic acid molecules are not related to the degeneracy of the genetic code.

To isolate the Src substrates of the invention, a screening strategy based on the in vitro phosphorylation of lambda expression libraries immobilized on nitrocellulose filters by Src containing Sf9 lysates was employed to isolate Src substrates, as in Lock, P. et. al. EMBO J. 17(15):4346-4357, 1998, which is incorporated in its entirety, including any drawings. Lambda clones encoding 4 Src substrates were identified in this manner, tks 107, 113, 118 and tks 202.

### Tks 107 (Grub)

The lambda clone tks 107 was isolated from a λZap cDNA library constructed with cDNA from the human lung tumor cell line NCI-H460. The DNA fragment encoding tks 107 was used to isolate various overlapping clones of tks 107 from the same library. One of these clones (tks 107-4) contains a large open reading frame (ORF) with an in frame stop codon upstream of a putative initiating methionine. This ORF is predicted to encode a protein of 1511 amino acids and has been renamed Grub. Computer searches of public sequence data bases with this sequence as a query revealed that this ORF contains a dbl homology domain (DH) followed by a pleckstrin homology domain (PH) in its C-terminus.

The dbl homology domain (DH domain) is a region of approximately 250 amino acids intially found in Dbl and the Cdc24 protein. Subsequently, a growing list (>20) of proteins have been found to contain DH domains. DH domains of different proteins share about 30% amino acid identity to each other. Proteins containing DH domains have been shown to act as Guanine Exchange Factors (GEF's) for the Rho members of small GTPases. Many DH containing proteins exhibit cellular transformation activity upon N-terminal truncation (where the DH domain is left unmutated). (Reviewed in Quilliam, L.A., et. al. BioEssays, 17:395-404, 1995. Whitehead, I.P., et. al., Biochimica et Biophysica Acta, 1332:F1-F23, 1997. Cerione, R.A. and Zheng, Y. Curr. Opin. Cell Biol. 8:216-222, 1996). Recently, the 3 dimensional structure of the DH domain of human Sos was elucidated (Soisson, S.M., et. al., Cell, 95:259-268). It was shown to be composed of α-helixes, several of which correspond well to previously defined conserved regions (CR) of DH domains (Whitehead, I.P., et. al., Biochimica et Biophysica Acta, 1332:F1-F23, 1997, and figure Grub GEF align). A hidden Markov model (HMM) for DH domain was built using HMMER (http://hmmer.wustl.edu). Input to HMMER was a multiple-sequence alignment generated by CLUSTALW (Thmpson, JD, Higgins DG, Gibson, TJ, Nucleic Acid Research, 1994). Secondary structural information from (Soisson, S.M., et. al., Cell, 95: 259-268) was incorporated into CLUSTALW alignment. The amino acid region from 1083-1257 of Seq LD. NO: 5 (Tks107/Grub) aligned well with this Hidden Markov Model, exhibiting both conserved α-helical motifs as well as conserved amino acid residues (Grub GEF align figure).

In addition, two EST's (AA778210 and AA829341) were found which overlapped the 3' sequence of tks 107-4 and extended the 3' untranslated sequence to the poly-adenylation tail. The sequence compiled from tks 107, tks 107-4 and the two EST's is shown in SEQ ID NO:1 and was renamed Grub. Proposed relevant features of this sequence include:

| nucleotide(s) | Comments |
|---|---|
| 155-1778 | sequence of original tks 107 |
| 1-5107 | sequence of tks 107-4 |
| 208-4764 | putative coding region of Grub |
| 3454-3978 | Dbl homology domain |
| 4000-4323 | Pleckstrin homology domain |
| 4164-5335 | EST AA778210 |
| ?-5332 | EST AA829341 (5' limit is unknown) |
| 5305-5315 | Not present in AA829341 |
| 4897 | T instead of G in the EST's |
| 5005 | T instead of G in the EST's |
| 5010 | T instead of G in the EST's |
| 5072 | T instead of C in the EST's |
| 5103 | T instead of C in the EST's |

The tandem array of DH/PH domains in the C-terminus of Grub is a hallmark of the Dbl family of proteins. Many members of this family exhibit cellular transformation activity upon N-terminal truncations. Cellular transformation requires that the DH/PH modules be intact. The DH/PH modules of many Dbl family proteins have been shown to regulate the activity of various Rho sub-family small GTPases by serving as Guanine Exchange Factors (GEF). Many Rho family GTPases have been shown to regulate the assembly of actin structures and may also regulate gene transcription through activation of various kinase pathways such as Jnk (Hall, A. Science, 279:509-514, 1998).

Grub mRNA is expressed in all human tissues examined, with particularly high expression in the pancreas and spleen. The expression level of Grub was determined for 60 tumor cell lines and was found to be significantly elevated in 13 (~21%) cell lines and may be slightly elevated in 10 or more cell lines. Deletion analyses show that Grub is phosphorylated on the N-terminus by Src when both are co-expressed in 293 cells. Recombinant Grub expressed in 293 cells migrates with an apparent Mr of ~190,000. Although this is larger than its predicted Mr of 164,701 (based on the primary amino acid sequence), it is often difficult to accurately determine Mr for proteins in this size range and it is unknown whether Grub contains any post-translational modifications that may alter its Mr.

NIH 3T3 clones stably expressing full length, N-terminus or C-terminus of Grub have been generated. These clones do not show enhanced growth rates, exhibit growth in soft agar, nor demonstrate altered morphologies (data not shown). However co-expression of the N or C terminus of Grub in 293 cells with HA-JNK activate Jnk activity 4-6X over that of vector alone. Co-expression of wild type Grub with HA-Jnk stimulates Jnk activity about 2.5X, despite the fact that wild type Grub is expressed at a level much lower (approximately 1/10) than either the N or C terminus of Grub. In contrast, excessive overexpression of another heterologous protein (SH3P7ΔSH3) only activates Jnk expression weakly. These data suggest that the C-term of Grub can activate Jnk activity possibly by acting as a guanine exchange factor for an unknown Rho family GTPase. It is interesting to note that the DH/PH or DH domain of Grub by themselves do not activate Jnk activity strongly. This is consistent with anecdotal evidence that the DH domains of some dbl family members require flanking region to exhibit full activity (perhaps due to additional structural stability conferred by the flanking sequences). The N-term is also capable of activating Jnk. While the mechanism by which the N-term activates Jnk is currently unknown, it is possible that the N-term contain domain or domains with enzymatic (i.e. a cryptic GEF activity) or binding activities toward proteins capable of activating Jnk.

To further our understanding of possible Grub function, the N-terminus of Grub was used as bait in a yeast 2-hybrid screen in order to isolate Grub interacting proteins. Approximately 1.8×10⁶ transformants of a Hela cdNA library were isolated. 6 independent cDNA's encoding the protein snapin and 2 independent cDNA's encoding the human homologue of the rat RACK1 were isolated in this assay.

Snapin is a small neuron specific protein, localized on synaptic vesicles which serves as a bridge between the SNARE complex and synaptotagmin (Ilardi, J., et. al., Nature Neuro. 1999, 2:119-124). Synaptotagmin is the calcium sensor that triggers the final fusion of ynaptic vesicles with the plasma membrame. Small GTPases of the Rab subfamily of GTPases also play a role in efficient synaptic transport (Sudhof, T.C., Nature 1996, 375: 645-653). It is tempting to hypothesize that Grub could act as a GEF for these small GTPases. Alternatively, Grub may act in a more traditional manner as a GEF for a Rho family GTPase, but by binding to snapin, links vesicular transport to the actin cycle by regulating Rho family members.

RACK1 was initially cloned as an intracellular receptor for activated Protein Kinase C (Ron, D., et. al., P.N.A.S. 1994, 91:839-843). Since then, RACK1 has been shown to bind to a variety of signalling molecules, including Src, and may act as a molecular scaffold on which signalling complexes may assemble (Rodriguez MM, *et al.* Biochemistry. 1999 38: 13787-94. Liliental, J. and Chang, D.D. JBC 1998 273: 2379-2383. Chang, B., *et al.,* MCB 1998 18:32453256.).

### Tks 113

The lambda clone tks 113 was isolated from a λZap cDNA library constructed with cDNA from the human lung tumor cell line NCI-H460. Tks contains a 1430 nucleotide EcoRIXhoI fragment. This fragment encodes an ORF of 476 amino acids with no significant homologies to any publicly available sequence. However, tks 113 contains several PXXP motifs in a C terminal proline rich domain (26% prolines), suggesting that it may be a binding partner for signalling molecules with SH3 domains. Proteins serving as scaffolds, anchors or adaptors for signalling complexes often display multiple motifs (such as PXXP) for protein/protein interactions (Pawson, T. and Scott, J.D., Science 278:2075-2080, 1997).

The sequence of Tks113 is shown in SEQ ID NO:2. The relevant features of this sequence include: Tks 113 mRNA is expressed in all human tissues examined and is most highly expressed in testis, heart and skeletal muscle. The apparent size of tks 113's mRNA in these experiments is approximately 2.4 kb. In addition, an antibody raised against the N-terminus of tks 113 identifies a protein of approximately 77 kDa in NIH 3T3 cells. Therefore, based on these data, tks 113 is unlikely to be a full length clone. Isolation of a full length clone is neccessary to allow generation of reagents such as expression constructs in order to elucidate the function of tks 113.

### Tks 118

Tks 118 was isolated from a λZap cDNA library constructed with cDNA from the human lung tumor cell line NCI-H460. Tks 118 is a clone of 1532 nucleotides with a large ORF of 430 amino acids (starting at the putative initiating methionine). An independent clone (tks 109), indentical to the first 1115 nucleotides of tks 118, was also isolated in the same screen. Searches of the public data base with these sequences revealed that tks 118 is 86% identical to a previously cloned murine sequence SH3P7, suggesting that tks 118 is likely to be the human orthologue of mSH3P7 (Sparks, et. al., Nature Biotech. 14:741-744, 1996). Furthermore, the N-terminal 240 amino acids of tks 118 is 44% identical to the chicken protein, Drebrin. Drebrin has been reported to bind and alter actin structures (Shirao, T., J. Biochem., 117:231-236, 1995). In addition, hSH3P7 also contains an SH3 domain in the C-terminal 62 amino acids. The sequence of tks 118 is shown in SEQ ID NO:3. The relevant features of this sequence include:

| nucleotides | comments |
|---|---|
| 1-1535 | Tks118 sequence |
| 1-1115 | tks109 sequence |
| 26-1319 | Tks 118 coding sequence |
| 1-720 | Drebrin homology domain |
| 1130-1319 | SH3 domain |
| 966 | T instead of C in tks 109 |
| 969 | T instead of C in tks 109 |
| 1023 | T instead of C in tks 109 |

Tks118 mRNA is expressed in all tissues examined, with highest expression in spleen, thymus, prostate and peripheral blood leukocytes. Tks118 mRNA is overexpressed in >40% of tumor cell lines tested. Tks118 is phosphorylated by Src upon co-expression in 293 cells. Overexpression of Tks118 in NIH 3T3 and Src transformed NIH 3T3 cells leads to localization at actin like structures. Noteably, Tks118 localizes to podosomes in Src transformed cells. This localization depends on the Drebrin homology domain and is consistent with previous reports that Drebrin binds and alter actin structures. Recently, it has been shown that m-SH3P7 is tyrosine phosphorylated in antigen receptor stimulated lymphocytes and co-localizes with actin structures in NIH 3T3 cells (Larbolette, O., MCB. 19:1539-1546, 1999)

Presently, we will determine if Tks118 binds and/or affect actin structures in vitro and in vivo. Overexpression of various Tks118 constructs in cell lines will be carried out to determine Tks118's effects on cell growth, transformation, and motility.

### Tks 202

The lambda clone tks 202 was isolated from a λZap cDNA library constructed with cDNA from the human colon tumor cell line Colo 205 (Stratagene). The DNA sequence encoding tks 202 was used to query public data bases to uncover any informative homologies. This search revealed that the 3' end of tks 202 encodes an amino acid sequence homologous to proteins of the small GTPase family. In addition, 2 EST's (AA470519 and r78655) were also found that overlapped with the 3' sequence of tks 202. Sequences from these ESTs were used to extend the tks 202 sequence to the poly-adenylation site. A 5' -500 nucleotide RI/XbaI fragment from Tks 202 was used to probe a Trip;Ex Prostate library (Clontech) to isolate additional Tks 202 clones. One of these clones, Tks 202-17 extended the 5' sequence by 933 nucleotides. An additional 370 nucleotides of 5' sequences was obtained by a modification of 5' RACE as described below. This 5' RACE product revealed a stop codon at nucleotide 170, 5' of a putative starting methionine at nucleotide 647. The sequence compiled from Tks202, Tks202-17, the two EST's, and the 5' RACE product is shown in SEQ ID NO:4. The relevant features of this sequence include:

| nucleotides | Comments |
|---|---|
| 1305-3155 | Tks202 |
| 371-1798 | Tks202-17 |
| 647-2866 | Putative Tks202 coding region |
| 2258-2866 | GTPase domain |
| 1-532 5' | RACE product |
| 1788-3155 | EST AA470519 |
| 2544-3155 | EST r78655 |

DNA fragments of tks 202 were used to probe multiple tissue northern blots (Clontech). This revealed that the endogeneous tks 202 mRNA is approximately 5 kb and is expressed at very low or undetectable levels in most tissues tested with the exception of prostate and pancreas.

Small GTPases represent a large family of proteins that act as molecular switches that control diverse biological functions, including cell proliferation and differentiation, cytoskeletal organization, protein transport, cell cycle and free radical production (Bourne, H.R. et. al., Nature, 348(6297):125-32, 1990. Bourne, H.R. et. al., Nature, 349(6305):117-27, 1991). That GTPases modulate these central cellular pathways suggest that both GTPases and their regulators are of critical importance.

Small GTPases are typically about 200 amino acids, almost all of which is folded into a single GTPase domain. As such, tks 202 is unusual in that it contains a significant extension N-terminal to its GTPase domain. One could speculate that the presence of this extension may implicate tks 202 (and as yet undiscovered GTPases of similar structures) in cellular roles previously unsuspected for GTPases or may represent a novel mechanism for regulating GTPase activity.

### II. Nucleic Acid Probes, Methods, and Kits for Detection of the Proteins of the Invention.

A nucleic acid probe of the present invention may be used to probe an appropriate chromosomal or cDNA library by usual hybridization methods to obtain other nucleic acid molecules of the present invention. A chromosomal DNA or cDNA library may be prepared from appropriate cells according to recognized methods in the art (cf. "Molecular Cloning: A Laboratory Manual", second edition, Cold Spring Harbor Laboratory, Sambrook, Fritsch, & Maniatis, eds., 1989).

In the alternative, chemical synthesis can be carried out in order to obtain nucleic acid probes having nucleotide sequences which correspond to N-terminal and C-terminal portions of the amino acid sequence of the polypeptide of interest. The synthesized nucleic acid probes may be used as primers in a polymerase chain reaction (PCR) carried out in accordance with recognized PCR techniques, essentially according to PCR Protocols, "A Guide to Methods and Applications", Academic Press, Michael, *et al.,* eds., 1990, utilizing the appropriate chromosomal or cDNA library to obtain the fragment of the present invention.

One skilled in the art can readily design such probes based on the sequence disclosed herein using methods of computer alignment and sequence analysis known in the art ("Molecular Cloning: A Laboratory Manual", 1989, supra). The hybridization probes of the present invention can be labeled by standard labeling techniques such as with a radiolabel, enzyme label, fluorescent label, biotin-avidin label, chemiluminescence, and the like. After hybridization, the probes may be visualized using known methods.

The nucleic acid probes of the present invention include RNA, as well as DNA probes, such probes being generated using techniques known in the art. The nucleic acid probe may be immobilized on a solid support. Examples of such solid supports include, but are not limited to, plastics such as polycarbonate, complex carbohydrates such as agarose and sepharose, and acrylic resins, such as polyacrylamide and latex beads. Techniques for coupling nucleic acid probes to such solid supports are well known in the art.

The test samples suitable for nucleic acid probing methods of the present invention include, for example, cells or nucleic acid extracts of cells, or biological fluids. The samples used in the above-described methods will vary based on the assay format, the detection method and the nature of the tissues, cells or extracts to be assayed. Methods for preparing nucleic acid extracts of cells are well known in the art and can be readily adapted in order to obtain a sample which is compatible with the method utilized.

One method of detecting the presence of nucleic acids of the invention in a sample comprises (a) contacting said sample with the above-described nucleic acid probe under conditions such that hybridization occurs and (b) detecting the presence of said probe bound to said nucleic acid molecule. One skilled in the art would select the nucleic acid probe according to techniques known in the art as described above. Samples to be tested include but should not be limited to RNA samples of human tissue.

A kit for detecting the presence of nucleic acids of the invention in a sample comprises at least one container means having disposed therein the above-described nucleic acid probe. The kit may further comprise other containers comprising one or more of the following: wash reagents and reagents capable of detecting the presence of bound nucleic acid probe. Examples of detection reagents include, but are not limited to radiolabelled probes, enzymatic labeled probes (horseradish peroxidase, alkaline phosphatase), and affinity labeled probes (biotin, avidin, or steptavidin).

In detail, a compartmentalized kit includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the test sample, a container which contains the probe or primers used in the assay, containers which contain wash reagents (such as phosphate buffered saline, Tris-buffers, and the like), and containers which contain the reagents used to detect the hybridized probe, bound antibody, amplified product, or the like. One skilled in the art will readily recognize that the nucleic acid probes described in the present invention can readily be incorporated into one of the established kit formats which are well known in the art.

### III. DNA Constructs Comprising a Tks 107, Tks 113, Tks 118 and Tks 202 Related Nucleic Acid Molecule and Cells Containing These Constructs.

The present invention also relates to a recombinant DNA molecule comprising, 5' to 3', a promoter effective to initiate transcription in a host cell and the above-described nucleic acid molecules. In addition, the present invention relates to a recombinant DNA molecule comprising a vector and an above-described nucleic acid molecule. The present invention also relates to a nucleic acid molecule comprising a transcriptional region functional in a cell, a sequence complementary to an RNA sequence encoding an amino acid sequence corresponding to the above-described polypeptide, and a transcriptional termination region functional in said cell. The above-described molecules may be isolated and/or purified DNA molecules.

The present invention also relates to a cell or organism that contains an above-described nucleic acid molecule and thereby is capable of expressing a polypeptide. The polypeptide may be purified from cells which have been altered to express the polypeptide. A cell is said to be "altered to express a desired polypeptide" when the cell, through genetic manipulation, is made to produce a protein which it normally does not produce or which the cell normally produces at lower levels. One skilled in the art can readily adapt procedures for introducing and expressing either genomic, cDNA, or synthetic sequences into either eukaryotic or prokaryotic cells.

A nucleic acid molecule, such as DNA, is said to be "capable of expressing" a polypeptide if it contains nucleotide sequences which contain transcriptional and translational regulatory information and such sequences are "operably linked" to nucleotide sequences which encode the polypeptide. An operable linkage is a linkage in which the regulatory DNA sequences and the DNA sequence sought to be expressed are connected in such a way as to permit gene sequence expression. The precise nature of the regulatory regions needed for gene sequence expression may vary from organism to organism, but shall in general include a promoter region which, in prokaryotes, contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence, CAAT sequence, and the like.

If desired, the non-coding region 3' to the sequence encoding a polypeptide of the invention may be obtained by the above-described methods. This region may be retained for its transcriptional termination regulatory sequences, such as termination and polyadenylation. Thus, by retaining the 3'-region naturally contiguous to the DNA sequence encoding a polypeptide of the invention, the transcriptional termination signals may be provided. Where the transcriptional termination signals are not satisfactorily functional in the expression host cell, then a 3' region functional in the host cell may be substituted.

Two DNA sequences (such as a promoter region sequence and a sequence encoding a polypeptide of the invention) are said to be operably linked if the nature of the linkage between the two DNA sequences does not (1) result in the introduction of a frame-shift mutation, (2) interfere with the ability of the promoter region sequence to direct the transcription of a gene sequence encoding a polypeptide of the invention or (3) interfere with the ability of the gene sequence of a polypeptide of the invention to be transcribed by the promoter region sequence. Thus, a promoter region would be operably linked to a DNA sequence if the promoter were capable of effecting transcription of that DNA sequence. Thus, to express a gene encoding a polypeptide of the invention, transcriptional and translational signals recognized by an appropriate host are necessary.

The present invention encompasses the expression of a gene encoding a polypeptide of the invention (or a functional derivative thereof) in either prokaryotic or eukaryotic cells. Prokaryotic hosts are, generally, very efficient and convenient for the production of recombinant proteins and are, therefore, one type of preferred expression system for polypeptides of the invention. Prokaryotes most frequently are represented by various strains of *E*. *coli.* However, other microbial strains may also be used, including other bacterial strains.

In prokaryotic systems, plasmid vectors that contain replication sites and control sequences derived from a species compatible with the host may be used. Examples of suitable plasmid vectors may include pBR322, pUC118, pUC119 and the like; suitable phage or bacteriophage vectors may include γgt10, γgt11 and the like; and suitable virus vectors may include pMAM-neo, pKRC and the like. Preferably, the selected vector of the present invention has the capacity to replicate in the selected host cell.

Recognized prokaryotic hosts include bacteria such as *E*. *coli, Bacillus, Streptomyces, Pseudomonas, Salmonella, Serratia,* and the like. However, under such conditions, the polypeptide will not be glycosylated. The prokaryotic host must be compatible with the replicon and control sequences in the expression plasmid.

To express a polypeptide of the invention (or a functional derivative thereof) in a prokaryotic cell, it is necessary to operably link the sequence encoding the polypeptide of the invention to a functional prokaryotic promoter. Such promoters may be either constitutive or, more preferably, regulatable (i.e., inducible or derepressible). Examples of constitutive promoters include the *int* promoter of bacteriophage λ, the *bla* promoter of the β-lactamase gene sequence of pBR322, and the cat promoter of the chloramphenicol acetyl transferase gene sequence of pPR325, and the like. Examples of inducible prokaryotic promoters include the major right and left promoters of bacteriophage λ (P_{L} and P_{R}), the *trp, recA, λacZ,* λac*I*, and *gal* promoters of *E*. *coli,* the α-amylase (Ulmanen *et al.,* J. Bacteriol. 162:176-182, 1985) and the ζ-28-specific promoters of *B. subtilis* (Gilman *et al.,* Gene Sequence 32:11-20, 1984), the promoters of the bacteriophages of *Bacillus* (Gryczan, In: The Molecular Biology of the Bacilli, Academic Press, Inc., NY, 1982), and *Streptomyces* promoters (Ward *et al.,* Mol. Gen. Genet. 203:468-478, 1986). Prokaryotic promoters are reviewed by Glick (Ind. Microbiot. 1:277-282, 1987), Cenatiempo (Biochimie 68:505-516, 1986), and Gottesman (Ann. Rev. Genet. 18:415-442, 1984).

Proper expression in a prokaryotic cell also requires the presence of a ribosome-binding site upstream of the gene sequence-encoding sequence. Such ribosome-binding sites are disclosed, for example, by Gold *et al.* (Ann. Rev. Microbiol. 35:365-404, 1981). The selection of control sequences, expression vectors, transformation methods, and the like, are dependent on the type of host cell used to express the gene. As used herein, "cell", "cell line", and "cell culture" may be used interchangeably and all such designations include progeny. Thus, the words "transformants" or "transformed cells" include the primary subject cell and cultures derived therefrom, without regard to the number of transfers. It is also understood that all progeny may not be precisely identical in DNA content, due to deliberate or inadvertent mutations. However, as defined, mutant progeny have the same functionality as that of the originally transformed cell.

Host cells which may be used in the expression systems of the present invention are not strictly limited, provided that they are suitable for use in the expression of the polypeptide of interest. Suitable hosts may often include eukaryotic cells. Preferred eukaryotic hosts include, for example, yeast, fungi, insect cells, mammalian cells either *in vivo,* or in tissue culture. Mammalian cells which may be useful as hosts include HeLa cells, cells of fibroblast origin such as VERO or CHO-K1, or cells of lymphoid origin and their derivatives. Preferred mammalian host cells include SP2/0 and J558L, as well as neuroblastoma cell lines such as IMR 332, which may provide better capacities for correct post-translational processing.

In addition, plant cells are also available as hosts, and control sequences compatible with plant cells are available, such as the cauliflower mosaic virus 35S and 19S, and nopaline synthase promoter and polyadenylation signal sequences. Another preferred host is an insect cell, for example the *Drosophila* larvae. Using insect cells as hosts, the *Drosophila* alcohol dehydrogenase promoter can be used (Rubin, Science 240:1453-1459, 1988). Alternatively, baculovirus vectors can be engineered to express large amounts of polypeptides of the invention in insect cells (Jasny, Science 238:1653, 1987; Miller *et al.,* In: Genetic Engineering, Vol. 8, Plenum, Setlow *et al.,* eds., pp. 277-297, 1986).

Any of a series of yeast expression systems can be utilized which incorporate promoter and termination elements from the actively expressed sequences coding for glycolytic enzymes that are produced in large quantities when yeast are grown in mediums rich in glucose. Known glycolytic gene sequences can also provide very efficient transcriptional control signals. Yeast provides substantial advantages in that it can also carry out post-translational modifications. A number of recombinant DNA strategies exist utilizing strong promoter sequences and high copy number plasmids which can be utilized for production of the desired proteins in yeast. Yeast recognizes leader sequences on cloned mammalian genes and secretes peptides bearing leader sequences (*i.e.*, pre-peptides). Several possible vector systems are available for the expression of polypeptides of the invention in a mammalian host.

A wide variety of transcriptional and translational regulatory sequences may be employed, depending upon the nature of the host. The transcriptional and translational regulatory signals may be derived from viral sources, such as adenovirus, bovine papilloma virus, cytomegalovirus, simian virus, or the like, where the regulatory signals are associated with a particular gene sequence which has a high level of expression. Alternatively, promoters from mammalian expression products, such as actin, collagen, myosin, and the like, may be employed. Transcriptional initiation regulatory signals may be selected which allow for repression or activation, so that expression of the gene sequences can be modulated. Of interest are regulatory signals which are temperature-sensitive so that by varying the temperature, expression can be repressed or initiated, or are subject to chemical (such as metabolite) regulation.

Expression of polypeptides of the invention in eukaryotic hosts requires the use of eukaryotic regulatory regions. Such regions will, in general, include a promoter region sufficient to direct the initiation of RNA synthesis. Preferred eukaryotic promoters include, for example, the promoter of the mouse metallothionein I gene sequence (Hamer *et al.,* J. Mol. Appl. Gen. 1:273-288, 1982); the TK promoter of Herpes virus (McKnight, Cell 31:355-365, 1982); the SV40 early promoter (Benoist *et al.,* Nature (London) 290:304-31, 1981); and the yeast gal4 gene sequence promoter (Johnston *et al.,* Proc. Natl. Acad. Sci. (USA) 79:6971-6975, 1982; Silver *et al.,* Proc. Natl. Acad. Sci. (USA) 81:5951-5955, 1984).

Translation of eukaryotic mRNA is initiated at the codon which encodes the first methionine. For this reason, it is preferable to ensure that the linkage between a eukaryotic promoter and a DNA sequence which encodes a polypeptide of the invention (or a functional derivative thereof) does not contain any intervening codons which are capable of encoding a methionine (i.e., AUG). The presence of such codons results either in the formation of a fusion protein (if the AUG codon is in the same reading frame as the polypeptide of the invention coding sequence) or a frame-shift mutation (if the AUG codon is not in the same reading frame as the polypeptide of the invention coding sequence).

A nucleic acid molecule encoding a polypeptide of the invention and an operably linked promoter may be introduced into a recipient prokaryotic or eukaryotic cell either as a nonreplicating DNA or RNA molecule, which may either be a linear molecule or, more preferably, a closed covalent circular molecule. Since such molecules are incapable of autonomous replication, the expression of the gene may occur through the transient expression of the introduced sequence. Alternatively, permanent expression may occur through the integration of the introduced DNA sequence into the host chromosome.

A vector may be employed which is capable of integrating the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotrophic host, biocide resistance, e.g., antibiotics, or heavy metals, such as copper, or the like. The selectable marker gene sequence can either be directly linked to the DNA gene sequences to be expressed, or introduced into the same cell by co-transfection. Additional elements may also be needed for optimal synthesis of mRNA. These elements may include splice signals, as well as transcription promoters, enhancers, and termination signals. cDNA expression vectors incorporating such elements include those described by Okayama (Mol. Cell. Biol. 3:280-, 1983).

The introduced nucleic acid molecule can be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Preferred prokaryotic vectors include plasmids such as those capable of replication in *E. coli* (such as, for example, pBR322, ColEI, pSC101, pACYC 184, πVX; "Molecular Cloning: A Laboratory Manual", 1989, supra). Bacillus plasmids include pC194, pC221, pT127, and the like (Gryczan, In: The Molecular Biology of the Bacilli, Academic Press, NY, pp. 307-329, 1982). Suitable *Streptomyces* plasmids include p1J101 (Kendall *et al.,* J. Bacteriol. 169:4177-4183, 1987), and streptomyces bacteriophages such as φC31 (Chater *et al.,* In: Sixth International Symposium on Actinomycetales Biology, Akademiai Kaido, Budapest, Hungary, pp. 45-54, 1986). *Pseudomonas* plasmids are reviewed by John *et al.* (Rev. Infect. Dis. 8:693-704, 1986), and Izaki (Jpn. J. Bacteriol. 33:729-742, 1978).

Preferred eukaryotic plasmids include, for example, BPV, vaccinia, SV40, 2-micron circle, and the like, or their derivatives. Such plasmids are well known in the art (Botstein *et al.,* Miami Wntr. Symp. 19:265-274, 1982; Broach, In: "The Molecular Biology of the Yeast Saccharomyces: Life Cycle and Inheritance", Cold Spring Harbor Laboratory, Cold Spring Harbor, NY, p. 445-470, 1981; Broach, Cell 28:203-204, 1982; Bollon *et al.,* J. Clin. Hematol. Oncol. 10:39-48, 1980; Maniatis, In: Cell Biology: A Comprehensive Treatise, Vol. 3, Gene Sequence Expression, Academic Press, NY, pp. 563-608, 1980).

Once the vector or nucleic acid molecule containing the construct(s) has been prepared for expression, the DNA construct(s) may be introduced into an appropriate host cell by any of a variety of suitable means, i.e., transformation, transfection, conjugation, protoplast fusion, electroporation, particle gun technology, calcium phosphate-precipitation, direct microinjection, and the like. After the introduction of the vector, recipient cells are grown in a selective medium, which selects for the growth of vector-containing cells. Expression of the cloned gene(s) results in the production of a polypeptide of the invention, or fragments thereof. This can take place in the transformed cells as such, or following the induction of these cells to differentiate (for example, by administration of bromodeoxyuracil to neuroblastoma cells or the like). A variety of incubation conditions can be used to form the peptide of the present invention. The most preferred conditions are those which mimic physiological conditions.

### IV. The Proteins of the Invention and Their Expression Patterns

A variety of methodologies known in the art can be utilized to obtain the polypeptides of the present invention. The polypeptides may be purified from tissues or cells that naturally produce the polypeptides. Alternatively, the above-described isolated nucleic acid fragments could be used to express the polypeptides of the invention in any organism. The samples of the present invention include cells, protein extracts or membrane extracts of cells, or biological fluids. The samples will vary based on the assay format, the detection method, and the nature of the tissues, cells or extracts used as the sample.

Any eukaryotic organism can be used as a source for the polypeptides of the invention, as long as the source organism naturally contains such polypeptides. As used herein, "source organism" refers to the original organism from which the amino acid sequence of the subunit is derived, regardless of the organism the subunit is expressed in and ultimately isolated from.

One skilled in the art can readily follow known methods for isolating proteins in order to obtain the polypeptides free of natural contaminants. These include, but are not limited to: size-exclusion chromatography, HPLC, ion-exchange chromatography, and immuno-affinity chromatography.

### Tks 107 (Grub)

The tandem array of DH/PH domains in the C-terminus of Grub is a hallmark of the Dbl family of proteins. Many members of this family exhibit cellular transformation activity upon N-terminal truncations. Cellular transformation requires that the DH/PH modules be intact.

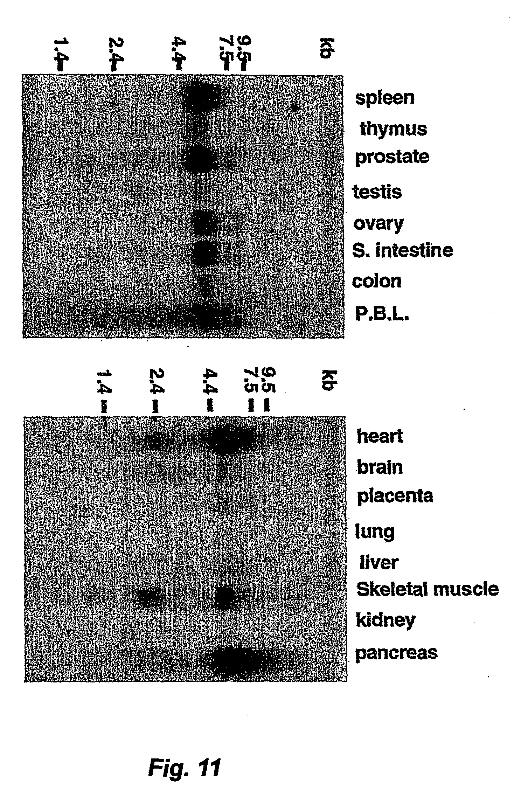

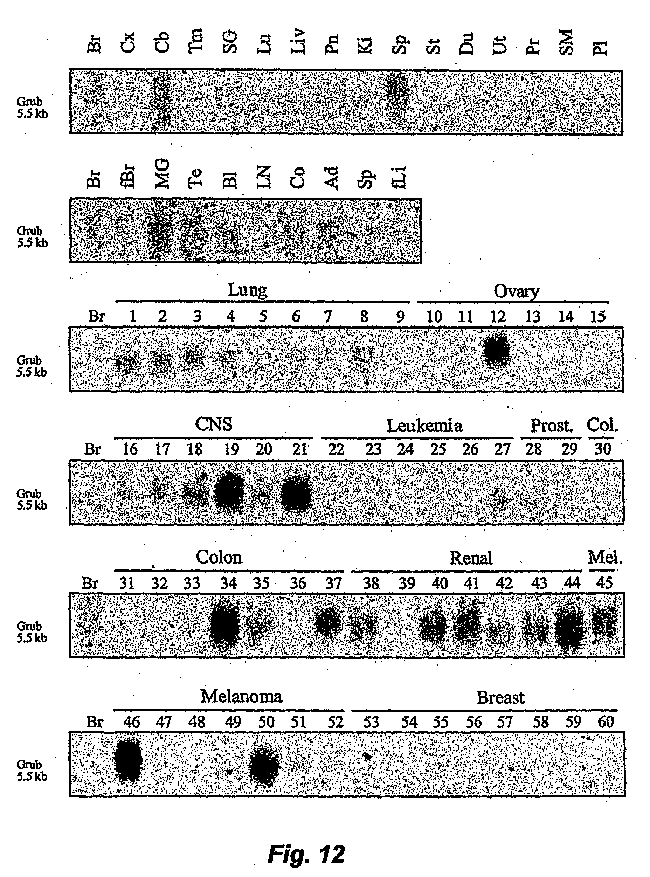

Grub mRNA is expressed in all human tissues examined, with particularly high expression in the pancreas and spleen (Fig. 11). The expression level of Grub was determined for 60 tumor cell lines and was found to be significantly elevated in 13 (~21%) cell lines and may be slightly elevated in 10 or more cell lines (Fig. 12). Deletion analyses show that Grub is phosphorylated on the N-terminus by Src when both are co-expressed in 293 cells (Fig. 13). Recombinant Grub expressed in 293 cells migrates with an apparent Mr of ~190,000 (Fig. 13). Although this is larger than its predicted Mr of 164,701 (based on the primary amino acid sequence), it is often difficult to accurately determine Mr for proteins in this size range and it is unknown whether Grub contains any post-translational modifications that may alter its Mr.

Characterization of the consequences of overexpression of normal and truncation mutants of Grub are underway, focusing particularly on whether they induce or reverse cellular transformation. Recombinantly expressed full length and truncated versions of Grub will be tested for their possible GEF activity toward various members of small GTPases. Stable clones will be isolated that express these recombinant proteins. They will be characterized for growth rate, DNA synthesis, cell-contact inhibition (foci formation), anchorage-independent growth (soft agar assays), and tumorigenicity in nude mice.

### Tks 113

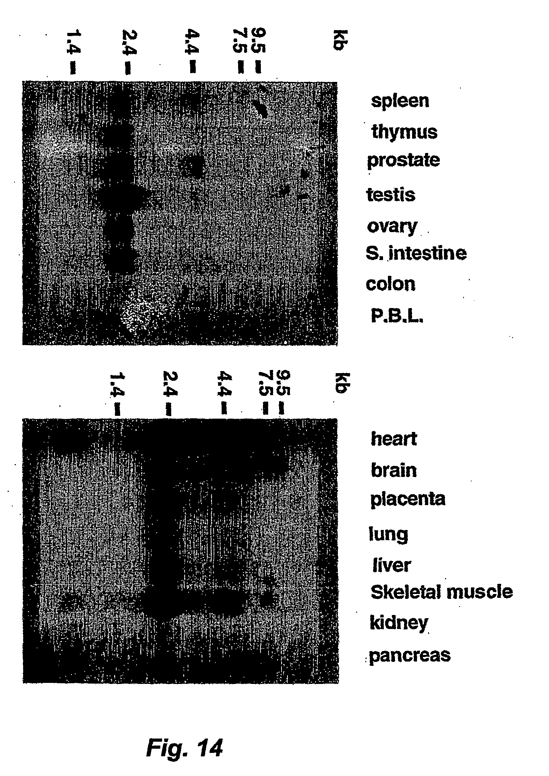

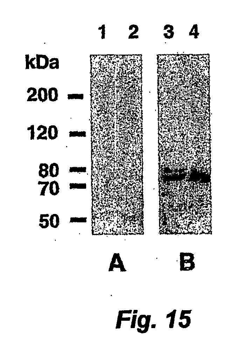

Tks 113 mRNA is expressed in all human tissues examined and is most highly expressed in testis, heart and skeletal muscle (Fig. 14). The apparent size of tks 113's mRNA in these experiments is approximately 2.4 kb. In addition, an antibody raised against the N-terminus of tks 113 identifies a protein of approximately 77 kDa in NIH 3T3 cells. Based on these data, tks 113 is unlikely to be a full length clone (Fig. 15).

### Tks 118

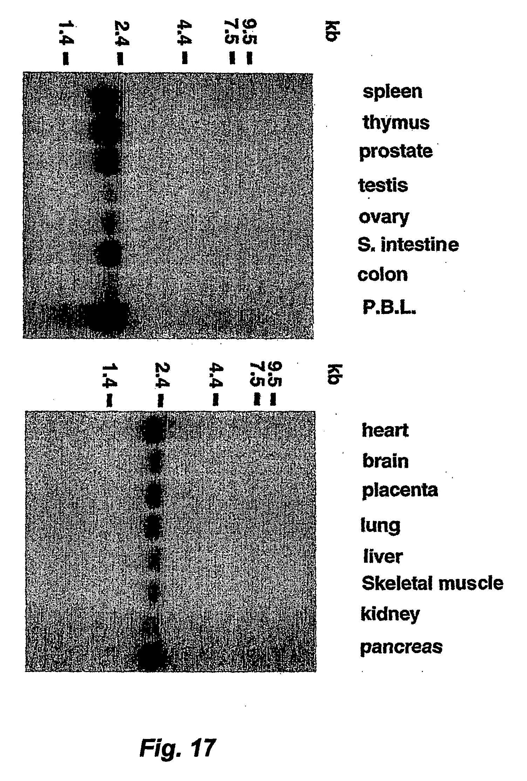

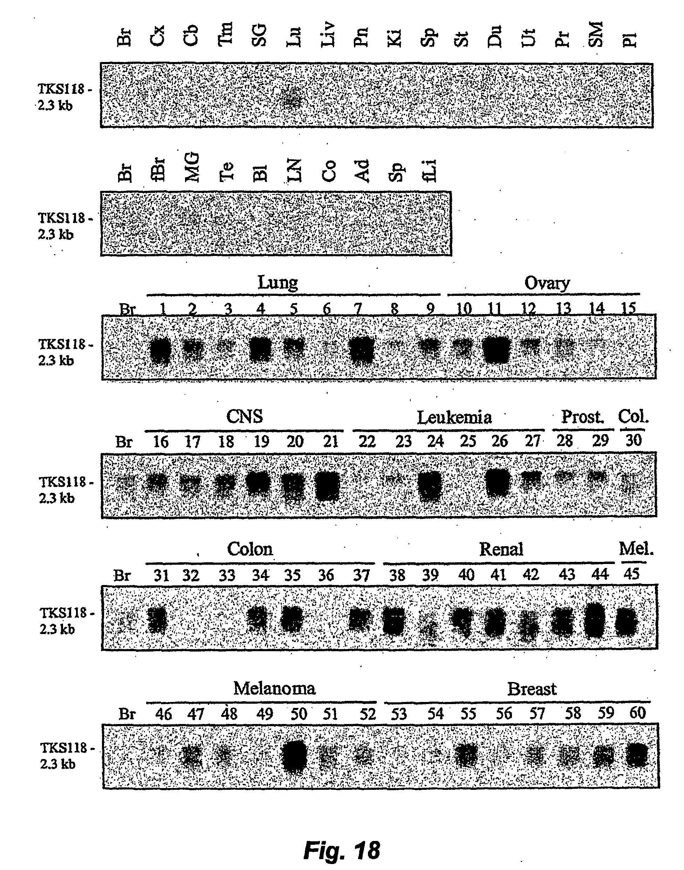

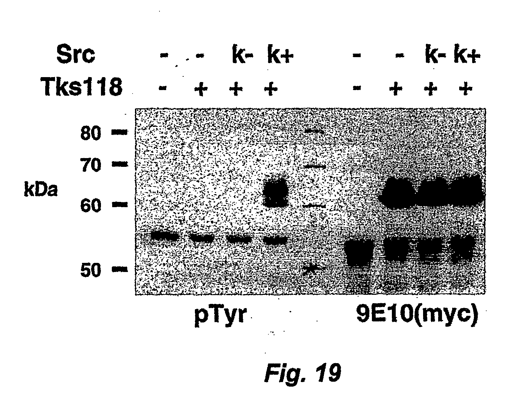

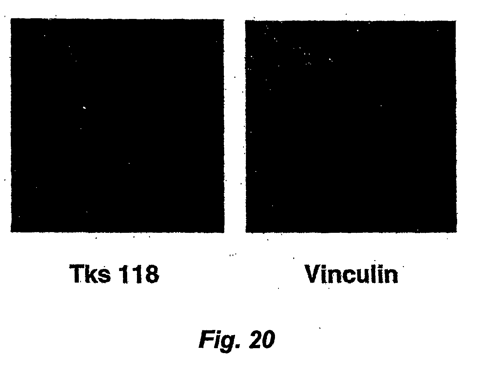

Tks mRNA is expressed in all tissues examined, with highest expression in spleen, thymus, prostate and peripheral blood leukocytes (Fig. 17). Tks 118 mRNA is overexpressed in >40% of tumor cell lines tested (Fig. 18). Tks 118 is phosphorylated by Src upon co-expression in 293 cells (Fig. 19). Overexpression of Tks 118 in NIH 3T3 and Src transformed NIH 3T3 cells leads to localization at actin like structures (Fig. 20). Noteably, Tks 118 localizes to podosomes in Src transformed cells (fig. 20). This localization depends on the Drebrin homology domain and is consistent with previous reports that Drebrin binds and alter actin structures. Recently, it has been shown that m-SH3P7 is tyrosine phosphorylated in antigen receptor stimulated lymphocytes and co-localizes with actin structures in NIH 3T3 cells (Larbolette, O., MCB. 19:1539-1546, 1999).

Presently, we will determine if Tks 118 binds and/or affect actin structures in vitro and in vivo. Overexpression of various Tks 118 constructs in cell lines will be carried out to determine Tks 118 effects on cell growth, transformation and motility.

### Tks 202

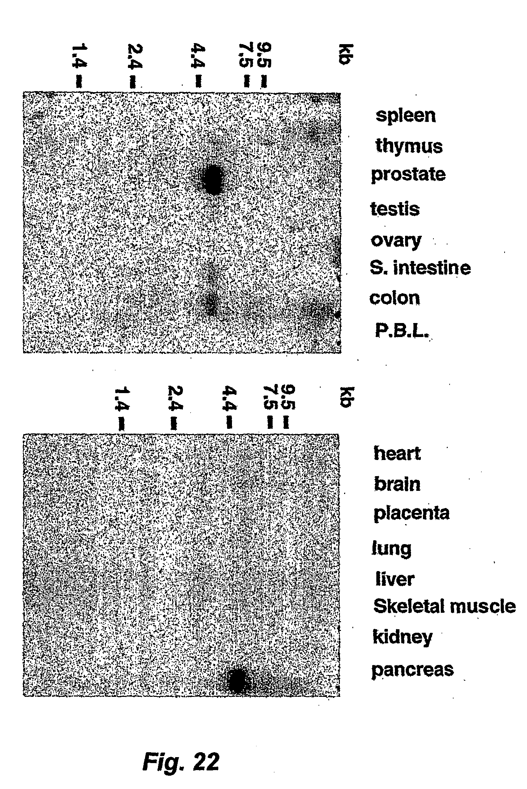

DNA fragments of tks 202 were used to probe multiple tissue northern blots (Clontech). This revealed that the endogeneous tks 202 mRNA is approximately 5 kb and is expressed at very low or undetectable levels in most tissues tested with the exception of prostate and pancreas (Fig. 22).

Small GTPases are typically about 200 amino acids, almost all of which is folded into a single GTPase domain. As such, tks 202 is unusual in that it contains a significant extension N-terminal to its GTPase domain. One could speculate that the presence of this extension may implicate Tks 202 (and as yet undiscovered GTPases of similar structures) in cellular roles previously unsuspected for GTPases or may represent a novel mechanism for regulating GTPase activity.

### V. Antibodies, Hybridomas, Methods of Use and Kits for Detection of Tks 107, Tks 113, Tks 118 or Tks 202

The present invention also relates to an antibody having specific binding affinity to a full or partial polypeptide of the invention. Such an antibody may be isolated by comparing its binding affinity to a polypetide of the invention with its binding affinity to other polypeptides. Those which bind selectively to a polypeptide of the invention would be chosen for use in methods requiring a distinction between a polypeptide of the invention and other polypeptides. Such methods could include, but should not be limited to, the analysis of altered expression in tissue containing other polypeptides.

The polypeptides of the present invention can be used in a variety of procedures and methods, such as for the generation of antibodies, for use in identifying pharmaceutical compositions, and for studying DNA/protein interaction.

The polypeptides of the present invention can be used to produce antibodies or hybridomas. One skilled in the art will recognize that if an antibody is desired, such a peptide could be generated as described herein and used as an immunogen. The antibodies of the present invention include monoclonal and polyclonal antibodies, as well fragments of these antibodies, and humanized forms. Humanized forms of the antibodies of the present invention may be generated using one of the procedures known in the art such as chimerization or CDR grafting.

The present invention also relates to a hybridoma which produces the above-described monoclonal antibody, or binding fragment thereof. A hybridoma is an immortalized cell line which is capable of secreting a specific monoclonal antibody.

In general, techniques for preparing monoclonal antibodies and hybridomas are well known in the art (Campbell, "Monoclonal Antibody Technology: Laboratory Techniques in Biochemistry and Molecular Biology," Elsevier Science Publishers, Amsterdam, The Netherlands, 1984; St. Groth *et al.,* J. Immunol. Methods 35:1-21, 1980). Any animal (mouse, rabbit, and the like) which is known to produce antibodies can be immunized with the selected polypeptide. Methods for immunization are well known in the art. Such methods include subcutaneous or intraperitoneal injection of the polypeptide. One skilled in the art will recognize that the amount of polypeptide used for immunization will vary based on the animal which is immunized, the antigenicity of the polypeptide and the site of injection.

The polypeptide may be modified or administered in an adjuvant in order to increase the peptide antigenicity. Methods of increasing the antigenicity of a polypeptide are well known in the art. Such procedures include coupling the antigen with a heterologous protein (such as globulin or β-galactosidase) or through the inclusion of an adjuvant during immunization.

For monoclonal antibodies, spleen cells from the immunized animals are removed, fused with myeloma cells, such as SP2/0-Ag14 myeloma cells, and allowed to become monoclonal antibody producing hybridoma cells. Any one of a number of methods well known in the art can be used to identify the hybridoma cell which produces an antibody with the desired characteristics. These include screening the hybridomas with an ELISA assay, western blot analysis, or radioimmunoassay (Lutz *et al*., Exp. Cell Res. 175:109-124, 1988). Hybridomas secreting the desired antibodies are cloned and the class and subclass are determined using procedures known in the art (Campbell, "Monoclonal Antibody Technology: Laboratory Techniques in Biochemistry and Molecular Biology", supra, 1984).

For polyclonal antibodies, antibody-containing antisera is isolated from the immunized animal and is screened for the presence of antibodies with the desired specificity using one of the above-described procedures. The above-described antibodies may be detectably labeled. Antibodies can be detectably labeled through the use of radioisotopes, affinity labels (such as biotin, avidin, and the like), enzymatic labels (such as horse radish peroxidase, alkaline phosphatase, and the like) fluorescent labels (such as FITC or rhodamine, and the like), paramagnetic atoms, and the like. Procedures for accomplishing such labeling are well-known in the art, for example, *see* Stemberger *et al.,* J. Histochem. Cytochem. 18:315,1970; Bayer *et al.,* Meth. Enzym. 62:308-, 1979; Engval *et al.,* Immunol. 109:129-, 1972; Goding, J. Immunol. Meth. 13:215-, 1976. The labeled antibodies of the present invention can be used for in vitro, in vivo and in situ assays to identify cells or tissues which express a specific peptide.

The above-described antibodies may also be immobilized on a solid support. Examples of such solid supports include plastics such as polycarbonate, complex carbohydrates such as agarose and sepharose, acrylic resins and such as polyacrylamide and latex beads. Techniques for coupling antibodies to such solid supports are well known in the art (Weir *et al.,* "Handbook of Experimental Immunology" 4th Ed., Blackwell Scientific Publications, Oxford, England, Chapter 10, 1986; Jacoby *et al.,* Meth. Enzym. 34, Academic Press, N.Y., 1974). The immobilized antibodies of the present invention can be used for *in vitro, in vivo* and *in situ* assays as well as in immunochromotography.

Furthermore, one skilled in the art can readily adapt currently available procedures, as well as the techniques, methods and kits disclosed herein with regard to antibodies, to generate peptides capable of binding to a specific peptide sequence in order to generate rationally designed antipeptide peptides (Hurby *et al.,* "Application of Synthetic Peptides: Antisense Peptides", In Synthetic Peptides, A User's Guide, W.H. Freeman, NY, pp. 289-307, 1992; Kaspczak *et al.,* Biochemistry 28:9230-9238, 1989).

Anti-peptide peptides can be generated by replacing the basic amino acid residues found in the peptide sequences of the polypeptides of the invention with acidic residues, while maintaining hydrophobic and uncharged polar groups. For example, lysine, arginine, and/or histidine residues are replaced with aspartic acid or glutamic acid and glutamic acid residues are replaced by lysine, arginine or histidine.

The present invention also encompasses a method of detecting a Tks 107, Tks 113, Tks 118 or Tks 202 polypeptide in a sample, comprising: (a) contacting the sample with an above-described antibody, under conditions such that immunocomplexes form, and (b) detecting the presence of said antibody bound to the polypeptide. In detail, the methods comprise incubating a test sample with one or more of the antibodies of the present invention and assaying whether the antibody binds to the test sample. Altered levels of a polypeptide of the invention in a sample as compared to normal levels may indicate disease.

Conditions for incubating an antibody with a test sample vary. Incubation conditions depend on the format employed in the assay, the detection methods employed and the type and nature of the antibody used in the assay. One skilled in the art will recognize that any one of the commonly available immunological assay formats (such as radioimmunoassays, enzyme-linked immunosorbent assays, diffusion based Ouchterlony, or rocket immunofluorescent assays) can readily be adapted to employ the antibodies of the present invention. Examples of such assays can be found in Chard ("An Introduction to Radioimmunoassay and Related Techniques" Elsevier Science Publishers, Amsterdam, The Netherlands, 1986), Bullock *et al.* ("Techniques in Immunocytochemistry," Academic Press, Orlando, FL Vol. 1, 1982; Vol. 2, 1983; Vol. 3, 1985), Tijssen ("Practice and Theory of Enzyme Immunoassays: Laboratory Techniques in Biochemistry and Molecular Biology," Elsevier Science Publishers, Amsterdam, The Netherlands, 1985).

The immunological assay test samples of the present invention include cells, protein or membrane extracts of cells, or biological fluids such as blood, serum, plasma or urine. The test samples used in the above-described method will vary based on the assay format, nature of the detection method and the tissues, cells or extracts used as the sample to be assayed. Methods for preparing protein extracts or membrane extracts of cells are well known in the art and can be readily adapted in order to obtain a sample which is testable with the system utilized.

A kit contains all the necessary reagents to carry out the previously described methods of detection. The kit may comprise: (i) a first container means containing an above-described antibody, and (ii) second container means containing a conjugate comprising a binding partner of the antibody and a label. In another preferred embodiment, the kit further comprises one or more other containers comprising one or more of the following: wash reagents and reagents capable of detecting the presence of bound antibodies.

Examples of detection reagents include, but are not limited to, labeled secondary antibodies, or in the alternative, if the primary antibody is labeled, the chromophoric, enzymatic, or antibody binding reagents which are capable of reacting with the labeled antibody. The compartmentalized kit may be as described above for nucleic acid probe kits. One skilled in the art will readily recognize that the antibodies described in the present invention can readily be incorporated into one of the established kit formats which are well known in the art.

### VI. Isolation of Compounds Which Interact With The Polypeptides of the Invention

The present invention also relates to a method of detecting a compound capable of binding to a Tks 107, Tks 113, Tks 118 or Tks 202 of the invention comprising incubating the compound with a Tks 107, Tks 113, Tks 118 or Tks 202 of the invention and detecting the presence of the compound bound to the Tks 107, Tks 113, Tks 118 or Tks 202. The compound may be present within a complex mixture, for example, serum, body fluid or cell extracts.

The present invention also relates to a method of detecting an agonist or antagonist of Tks 107, Tks 113, Tks 118 or Tks 202 activity or binding partner, such as src, activity comprising incubating cells that produce a polypeptide of the invention in the presence of a compound and detecting changes in the level of polypeptide activity or polypeptide binding partner activity. The compounds thus identified would produce a change in activity indicative of the presence of the compound. The compound may be present within a complex mixture, for example, serum, body fluid, or cell extracts. Once the compound is identified it can be isolated using techniques well known in the art.

The present invention also encompasses a method of agonizing (stimulating) or antagonizing a src-binding partner associated activity in a mammal comprising administering to said mammal an agonist or antagonist to a Tks 107, Tks 113, Tks 118 and Tks 202 polypeptide in an amount sufficient to effect said agonism or antagonism. A method of treating diseases in a mammal with an agonist or antagonist of Tks 107, Tks 113, Tks 118 or Tks 202 activity comprising administering the agonist or antagonist to a mammal in an amount sufficient to agonize or antagonize Tks 107, Tks 113, Tks 118 or Tks 202 associated functions is also encompassed in the present application.

In an effort to discover novel treatments for diseases, biomedical researchers and chemists have designed, synthesized, and tested molecules that inhibit the function of protein polypeptides. Some small organic molecules form a class of compounds that modulate the function of protein polypeptides. Examples of molecules that have been reported to inhibit the function of protein kinases include, but are not limited to, bis monocyclic, bicyclic or heterocyclic aryl compounds (PCT WO 92/20642, published November 26,1992 by Maguire *et al*.), vinylene-azaindole derivatives (PCT WO 94/14808, published July 7, 1994 by Ballinari et *al*.), 1-cyclopropyl-4-pyridyl-quinolones (U.S. Patent No. 5,330,992), styryl compounds (U.S. Patent No. 5,217,999), styryl-substituted pyridyl compounds (U.S. Patent No. 5,302,606), certain quinazoline derivatives (EP Application No. 0 566 266 A1), seleoindoles and selenides (PCT WO 94/03427, published February 17, 1994 by Denny *et al.),* tricyclic polyhydroxylic compounds (PCT WO 92/21660, published December 10, 1992 by Dow), and benzylphosphonic acid compounds (PCT WO 91/15495, published October 17, 1991 by Dow *et al),* all of which are incorporated by reference herein, including any drawings.

Compounds that can traverse cell membranes and are resistant to acid hydrolysis are potentially advantageous as therapeutics as they can become highly bioavailable after being administered orally to patients. However; many of these protein inhibitors only weakly inhibit function. In addition, many inhibit a variety of protein kinases and will therefore cause multiple side-effects as therapeutics for diseases.

Some indolinone compounds, however, form classes of acid resistant and membrane permeable organic molecules. WO 96/22976 (published August 1, 1996 by Ballinari *et al*.) describes hydrosoluble indolinone compounds that harbor tetralin, naphthalene, quinoline, and indole substituents fused to the oxindole ring. These bicyclic substituents are in turn substituted with polar groups including hydroxylated alkyl, phosphate, and ether substituents. U.S. Patent Application Serial Nos. 08/702,232, filed August 23, 1996, entitled "Indolinone Combinatorial Libraries and Related Products and Methods for the Treatment of Disease" by Tang *et al.* (Lyon & Lyon Docket No. 221/187) and 08/485,323, filed June 7, 1995, entitled "Benzylidene-Z-Indoline Compounds for the Treatment of Disease" by Tang *et al.* (Lyon & Lyon Docket No. 223/298) and International Patent Publication WO 96/22976, published August 1, 1996 by Ballinari *et al.,* all of which are incorporated herein by reference in their entirety, including any drawings, describe indolinone chemical libraries of indolinone compounds harboring other bicyclic moieties as well as monocyclic moieties fused to the oxindole ring. Applications 08/702,232, filed August 23, 1996, entitled "Indolinone Combinatorial Libraries and Related Products and Methods for the Treatment of Disease" by Tang *et al.* (Lyon & Lyon Docket No. 221/187), 08/485,323, filed June 7, 1995, entitled "Benzylidene-Z-Indoline Compounds for the Treatment of Disease" by Tang *et al.* (Lyon & Lyon Docket No. 223/298), and WO 96/22976, published August 1, 1996 by Ballinari *et al*. teach methods of indolinone synthesis, methods of testing the biological activity of indolinone compounds in cells, and inhibition patterns of indolinone derivatives, both of which are incorporated by reference herein, including any drawings.

Other examples of substances capable of modulating kinase activity include, but are not limited to, tyrphostins, quinazolines, quinoxolines, and quinolines. The quinazolines, tyrphostins, quinolines, and quinoxolines referred to above include well known compounds such as those described in the literature. For example, representative publications describing quinazolines include Barker *et al.,* EPO Publication No. 0 520 722 A1; Jones *et al.,* U.S. Patent No. 4,447,608; Kabbe *et al.,* U.S. Patent No. 4,757,072; Kaul and Vougioukas, U.S. Patent No. 5, 316,553; Kreighbaum and Comer, U.S. Patent No. 4,343,940; Pegg and Wardleworth, EPO Publication No. 0 562 734 A1; Barker *et al.,* Proc. of Am. Assoc, for Cancer Research 32:327 (1991); Bertino, J.R., Cancer Research 3:293-304 (1979); Bertino, J.R., Cancer Research 9(2 part 1):293-304 (1979); Curtin *et al.,* Br. J. Cancer 53:361-368 (1986); Fernandes *et* al., Cancer Research 43:1117-1123 (1983); Ferris *et al.* J. Org. Chem. 44(2):173-178; Fry *et al.,* Science 265:1093-1095 (1994); Jaclanan *et al.,* Cancer Research 51:5579-5586 (1981); Jones *et al.* J. Med. Chem. 29(6):1114-1118; Lee and Skibo, Biochemistry 26(23):7355-7362 (1987); Lemus *et al.,* J. Org. Chem. 54:3511-3518 (1989); Ley and Seng, Synthesis 1975:415-522 (1975); Maxwell *et al.,* Magnetic Resonance in Medicine 17:189-196 (1991); Mini *et al.,* Cancer Research 45:325-330 (1985); Phillips and Castle, J. Heterocyclic Chem. 17(19):1489-1596 (1980); Reece *et al.,* Cancer Research 47(11):2996-2999 (1977); Sculier *et al.,* Cancer Immunol. and Immunother. 23:A65 (1986); Sikora *et al*., Cancer Letters 23:289-295 (1984); and Sikora *et al.,* Analytical Biochem. 172:344-355 (1988), all of which are incorporated herein by reference in their entirety, including any drawings.

Quinoxaline is described in Kaul and Vougioukas, U.S. Patent No. 5,316,553, incorporated herein by reference in its entirety, including any drawings.

Quinolines are described in Dolle *et al.,* J. Med. Chem. 37:2627-2629 (1994); MaGuire, J. Med. Chem. 37:2129-2131 (1994); Burke *et al.,* J. Med. Chem. 36:425-432 (1993); and Burke *et al.* BioOrganic Med. Chem. Letters 2:1771-1774 (1992), all of which are incorporated by reference in their entirety, including any drawings.

Tyrphostins are described in *Allen et al.,* Clin. Exp. Immunol. 91:141-156 (1993); Anafi *et al.,* Blood 82:12:3524-3529 (1993); Baker *et al.,* J. Cell Sci. 102:543-555 (1992); Bilder *et al.,* Amer. Physiol. Soc. pp. 6363-6143:C721-C730 (1991); Brunton *et al.,* Proceedings of Amer. Assoc. Cancer Rsch. 33:558 (1992); Bryckaert *et al.,* Experimental Cell Research 199:255-261 (1992); Dong *et al.,* J. Leukocyte Biology 53:53-60 (1993); Dong *et al.,* J. Immunol. 151(5):2717-2724 (1993); Gazit *et al*., J. Med. Chem. 32:2344-2352 (1989); Gazit *et al.,* "J. Med. Chem. 36:3556-3564 (1993); Kaur *et al.,* Anti-Cancer Drugs 5:213-222 (1994); Kaur *et al.,* King *et* al., Biochem. J. 275:413-418 (1991); Kuo *et al.,* Cancer Letters 74:197-202 (1993); Levitzki, A., The FASEB J. 6:3275-3282 (1992); Lyall *et al.,* J. Biol. Chem. 264:14503-14509 (1989); Peterson *et al.,* The Prostate 22:335-345 (1993); Pillemer *et al.,* Int. J. Cancer 50:80-85 (1992); Posner *et al.,* Molecular Pharmacology 45:673-683 (1993); Rendu *et al.,* Biol. Pharmacology 44(5):881-888 (1992); Sauro and Thomas, Life Sciences 53:371-376 (1993); Sauro and Thomas, J. Pharm. and Experimental Therapeutics 267(3):119-1125 (1993); Wolbring *et al.,* J. Biol. Chem. 269(36):22470-22472 (1994); and Yoneda *et al.,* Cancer Research 51:4430-4435 (1991); all of which are incorporated herein by reference in their entirety, including any drawings.

Other compounds that could be used as modulators include oxindolinones such as those described in U.S. patent application Serial No. 08/702,232 filed August 23, 1996, incorporated herein by reference in its entirety, including any drawings.

### VIII. Transgenic Animals.

A variety of methods are available for the production of transgenic animals associated with this invention. DNA can be injected into the pronucleus of a fertilized egg before fusion of the male and female pronuclei or injected into the nucleus of an embryonic cell (*e.g*., the nucleus of a two-cell embryo) following the initiation of cell division (Brinster *et al.,* Proc. Nat. Acad. Sci. USA 82: 4438-4442, 1985). Embryos can be infected with viruses, especially retroviruses, modified to carry inorganic-ion receptor nucleotide sequences of the invention.

Pluripotent stem cells derived from the inner cell mass of the embryo and stabilized in culture can be manipulated in culture to incorporate nucleotide sequences of the invention. A transgenic animal can be produced from such cells through implantation into a blastocyst that is implanted into a foster mother and allowed to come to term. Animals suitable for transgenic experiments can be obtained from standard commercial sources such as Charles River (Wilmington, MA), Taconic (Germantown, NY), Harlan Sprague Dawley (Indianapolis, IN), etc.

The procedures for manipulation of the rodent embryo and for microinjection of DNA into the pronucleus of the zygote are well known to those of ordinary skill in the art (Hogan *et al.,* supra). Microinjection procedures for fish, amphibian eggs and birds are detailed in Houdebine and Chourrout (Experientia 47: 897-905, 1991). Other procedures for introduction of DNA into tissues of animals are described in U.S. Patent No., 4,945,050 (Sandford *et al.,* July 30, 1990).

By way of example only, to prepare a transgenic mouse, female mice are induced to superovulate. Females are placed with males, and the mated females are sacrificed by CO2 asphyxiation or cervical dislocation and embryos are recovered from excised oviducts. Surrounding cumulus cells are removed. Pronuclear embryos are then washed and stored until the time of injection. Randomly cycling adult female mice are paired with vasectomized males. Recipient females are mated at the same time as donor females. Embryos then are transferred surgically. The procedure for generating transgenic rats is similar to that of mice (Hammer *et al.,* Cell.63:1099-1112, 1990).

Methods for the culturing of embryonic stem (ES) cells and the subsequent production of transgenic animals by the introduction of DNA into ES cells using methods such as electroporation, calcium phosphate/DNA precipitation and direct injection also are well known to those of ordinary skill in the art (Teratocarcinomas and Embryonic Stem Cells, A Practical Approach, E.J. Robertson, ed., IRL Press, 1987).

In cases involving random gene integration, a clone containing the sequence(s) of the invention is co-transfected with a gene encoding resistance. Alternatively, the gene encoding neomycin resistance is physically linked to the sequence(s) of the invention. Transfection and isolation of desired clones are carried out by any one of several methods well known to those of ordinary skill in the art (E.J. Robertson, supra).

DNA molecules introduced into ES cells can also be integrated into the chromosome through the process of homologous recombination (Capecchi, Science 244: 1288-1292, 1989). Methods for positive selection of the recombination event (i.e., neo resistance) and dual positive-negative selection (i.e., neo resistance and gancyclovir resistance) and the subsequent identification of the desired clones by PCR have been described by Capecchi, supra and Joyner *et al.* (Nature 338: 153-156, 1989), the teachings of which are incorporated herein in their entirety including any drawings. The final phase of the procedure is to inject targeted ES cells into blastocysts and to transfer the blastocysts into pseudopregnant females. The resulting chimeric animals are bred and the offspring are analyzed by Southern blotting to identify individuals that carry the transgene. Procedures for the production of non-rodent mammals and other animals have been discussed by others (Houdebine and Chourrout, supra; Pursel *et al.,* Science 244:1281-1288, 1989; and Simms *et al*., Bio/Technology 6:179-183, 1988).

The invention provides transgenic, nonhuman mammals containing a transgene encoding a polypeptide of the invention or a gene effecting the expression of the polypeptide. Such transgenic nonhuman mammals are particularly useful as an *in vivo* test system for studying the effects of introduction of a polypeptide, or regulating the expression of a polypeptide (*i.e*., through the introduction of additional genes, antisense nucleic acids, or ribozymes).

All of the above references are incorporated by reference herein, including any drawings.

A "transgenic animal" is an animal having cells that contain DNA which has been artificially inserted into a cell, which DNA becomes part of the genome of the animal which develops from that cell. Preferred transgenic animals are primates, mice, rats, cows, pigs, horses, goats, sheep, dogs and cats. The transgenic DNA may encode human Tks 107, Tks 113, Tks 118 or Tks 202. Native expression in an animal may be reduced by providing an amount of anti-sense RNA or DNA effective to reduce expression of the receptor.

### IX. Gene Therapy

Tks 107, Tks 113, Tks 118 or Tks 202 genes or their genetic sequences will also be useful in gene therapy (reviewed in Miller, Nature 357:455-460,1992). Miller states that advances have resulted in practical approaches to human gene therapy that have demonstrated positive initial results. The basic science of gene therapy is described in Mulligan (Science 260:926-931, 1993).

In one preferred embodiment, an expression vector containing a Tks 107, Tks 113, Tks 118 or Tks 202 coding sequence is inserted into cells, the cells are grown *in vitro* and then infused in large numbers into patients. In another preferred embodi-ment, a DNA segment containing a promoter of choice (for example a strong promoter) is transferred into cells containing an endogenous gene of the invention in such a manner that the promoter segment enhances expression of the endogenous gene (for example, the promoter segment is transferred to the cell such that it becomes directly linked to the endogenous gene).

The gene therapy may involve the use of an adenovirus containing polypeptide cDNA targeted to a tumor, systemic polypeptide increase by implantation of engineered cells, injection with polypeptide-encoding virus, or injection of naked DNA into appropriate tissues.

Target cell populations may be modified by introducing altered forms of one or more components of the protein complexes in order to modulate the activity of such complexes. For example, by reducing or inhibit-ing a complex component activity within target cells, an abnormal signal transduction event(s) leading to a condition may be decreased, inhibited, or reversed. Deletion or missense mutants of a component, that retain the ability to interact with other components of the protein complexes but cannot function in signal transduction may be used to inhibit an abnormal, deleterious signal transduction event.

Expression vectors derived from viruses such as retroviruses, vaccinia virus, adenovirus, adeno-associ-ated virus, herpes viruses, several RNA viruses or bovine papilloma virus, may be used for delivery of nucleotide sequences (*e.g.*, cDNA) encoding recom-binant polypeptide of the invention protein into the targeted cell population (e.g., tumor cells). Methods which are well known to those skilled in the art can be used to construct recombinant viral vectors contain-ing coding sequences (Maniatis et *al.,* Molecu-lar Cloning: A Laboratory Manual, Cold Spring Harbor Laboratory, N.Y., 1989; Ausubel *et al*., Current Proto-cols in Molecular Biology, Greene Publishing Associates and Wiley Interscience, N.Y., 1989). Alter-natively, recombinant nucleic acid molecules encoding protein sequences can be used as naked DNA or in a recon-stituted system e.g., liposomes or other lipid systems for delivery to target cells (*e.g.*, Felgner *et al.,* Nature 337:387-8, 1989). Several other methods for the direct transfer of plasmid DNA into cells exist for use in human gene therapy and involve targeting the DNA to receptors on cells by complexing the plasmid DNA to proteins (Miller, supra).

In its simplest form, gene transfer can be performed by simply injecting minute amounts of DNA into the nucleus of a cell, through a process of microinjection (Capecchi, Cell 22:479-88, 1980). Once recombinant genes are introduced into a cell, they can be recognized by the cell's normal mechanisms for transcription and translation, and a gene product will be expressed. Other methods have also been attempted for introducing DNA into larger numbers of cells. These methods include: transfection, wherein DNA is precipitated with CaPO4 and taken into cells by pinocytosis (Chen *et al.,* Mol. Cell Biol. 7:2745-52, 1987); electroporation, wherein cells are exposed to large voltage pulses to introduce holes into the membrane (Chu *et al.,* Nucleic Acids Res. 15:1311-26, 1987); lipofection/liposome fusion, wherein DNA is packaged into lipophilic vesicles which fuse with a target cell (Felgner *et al.,* Proc. Natl. Acad. Sci. USA. 84:7413-7417, 1987); and particle bombardment using DNA bound to small projectiles (Yang *et al.,* Proc. Natl. Acad. Sci. 87:9568-9572, 1990). Another method for introducing DNA into cells is to couple the DNA to chemically modified proteins.

It has also been shown that adenovirus proteins are capable of destabilizing endosomes and enhancing the uptake of DNA into cells. The admixture of adenovirus to solutions containing DNA complexes, or the binding of DNA to polylysine covalently attached to adenovirus using protein crosslinking agents substantially improves the uptake and expression of the recombinant gene (Curiel *et al.,* Am. J. Respir. Cell. Mol. Biol., 6:247-52,1992).

As used herein "gene transfer" means the process of introducing a foreign nucleic acid molecule into a cell. Gene transfer is commonly performed to enable the expression of a particular product encoded by the gene. The product may include a protein, polypeptide, anti-sense DNA or RNA, or enzymatically active RNA. Gene transfer can be performed in cultured cells or by direct administration into animals. Generally gene transfer involves the process of nucleic acid contact with a target cell by non-specific or receptor mediated interactions, uptake of nucleic acid into the cell through the membrane or by endocytosis and release of nucleic acid into the cytoplasm from the plasma membrane or endosome. Expression may require, in addition, movement of the nucleic acid into the nucleus of the cell and binding to appropriate nuclear factors for transcription.

As used herein "gene therapy" is a form of gene transfer and is included within the definition of gene transfer as used herein and specifically refers to gene transfer to express a therapeutic product from a cell *in vivo* or *in vitro.* Gene transfer can be performed ex vivo on cells which are then transplanted into a patient, or can be performed by direct administration of the nucleic acid or nucleic acid-protein complex into the patient.

In another preferred embodiment, a vector having nucleic acid sequences encoding a Tks 107, Tks 113, Tks 118 or Tks 202 polypeptide is provided in which the nucleic acid sequence is expressed only in specific tissue. Methods of achieving tissue-specific gene expres-sion are set forth in International Publication No. WO 93/09236, filed November 3, 1992 and published May 13,1993.

All of the above-mentioned references are incorporated by reference herein, including any drawings.

In all of the preceding vectors set forth above, a further aspect of the invention is that the nucleic acid sequence contained in the vector may include additions, deletions or modifications to some or all of the sequence of the nucleic acid, as defined above.

In another preferred embodiment, a method of gene replacement is set forth. "Gene replacement" as used herein means supplying a nucleic acid sequence which is capable of being expressed *in vivo* in an animal and thereby providing or augmenting the function of an endogenous gene which is missing or defective in the animal.

### X. Administration of Substances

Methods of determining the dosages of compounds to be administered to a patient and modes of administering compounds to an organism are disclosed in U.S. Application Serial No. 08/702,282, filed August 23, 1996 and International patent publication number WO 96/22976, published August 1 1996, both of which are incorporated herein by reference in their entirety, including any drawings, figures or tables. Those skilled in the art will appreciate that such descriptions are applicable to the present invention and can be easily adapted to it.

The proper dosage depends on various factors such as the type of disease being treated, the particular composi-tion being used and the size and physiological condition of the patient. Therapeutically effective doses for the compounds described herein can be estimated initially from cell culture and animal models. For example, a dose can be formulated in animal models to achieve a circulating concentration range that initially takes into account the IC₅₀ as determined in cell culture assays. The animal model data can be used to more accurately determine useful doses in humans.

Plasma half-life and biodistribution of the drug and metabolites in the plasma, tumors and major organs can also be determined to facilitate the selection of drugs most appropriate to inhibit a disorder. Such measure-ments can be carried out. For example, HPLC analysis can be performed on the plasma of animals treated with the drug and the location of radiolabeled compounds can be deter-mined using detection methods such as X-ray, CAT scan and MRI. Compounds that show potent inhibitory activity in the screening assays, but have poor pharmacokinetic characteristics, can be optimized by altering the chemical structure and retesting. In this regard, compounds displaying good pharmacokinetic characteristics can be used as a model.

Toxicity studies can also be carried out by measuring the blood cell composition. For example, toxicity studies can be carried out in a suitable animal model as follows: 1) the compound is administered to mice (an untreated control mouse should also be used); 2) blood samples are periodically obtained via the tail vein from one mouse in each treatment group; and 3) the samples are analyzed for red and white blood cell counts, blood cell composition and the percent of lymphocytes versus polymorphonuclear cells. A comparison of results for each dosing regime with the controls indicates if toxicity is present.

At the termination of each toxicity study, further studies can be carried out by sacrificing the animals (preferably, in accordance with the American Veterinary Medical Association guidelines Report of the American Veterinary Medical Assoc. Panel on Euthanasia, *Journal of American Veterinary Medical Assoc.,* 202:229-249, 1993). Representative animals from each treatment group can then be examined by gross necropsy for immediate evidence of metastasis, unusual illness or toxicity. Gross abnormalities in tissue are noted and tissues are examined histologically. Compounds causing a reduction in body weight or blood components are less preferred, as are compounds having an adverse effect on major organs. In general, the greater the adverse effect the less preferred the compound.

For the treatment of cancers the expected daily dose of a hydrophobic pharmaceutical agent is between 1 to 500 mg/day, preferably 1 to 250 mg/day, and most preferably 1 to 50 mg/day. Drugs can be delivered less frequently provided plasma levels of the active moiety are sufficient to maintain therapeutic effectiveness.

Plasma levels should reflect the potency of the drug. Generally, the more potent the compound the lower the plasma levels necessary to achieve efficacy.

### EXAMPLES

The examples below are not limiting and are merely representative of various aspects and features of the present invention.

### Phosphorylation screening

The phosphorylation screening method we used to identify Src substrates was previously reported in Lock, P., et. al. EMBO J. 17(15):4346-4357, 1998). Approximately 1.2 X 10⁶ plaques from an NCI H460 λZAP library and ~4.9 x 10⁵ plaques from a Colo205 λZAPII library (Stratagene) were plated on 150 mm petri dishes. Plates were overlaid with nitrocellulose filters impregnated with 10 mM isopropyl-β-D-thiogalactopyranoside (IPTG) and incubated at 37 °C for 16 hours to induce expression of recombinant lacZ fusion proteins. Replica filters were washed extensively in TBST (10 mM Tris-HCl pH7.4, 150 mM NaCl, 0.1% Triton X-100) then equilibrated for one hour in kinase buffer (TBST containing 10 mM MgCl₂ and 2 mM MnCl₂). Filters were blocked for 1 hour at room temperature in kinase buffer containing 3% bovine serum albumin (BSA). Recombinant proteins were phosphorylated by incubating filters at 30 °C for 60 minutes in kinase buffer supplemented with 1/10 or 1/20 volume of an Sf9 extract containing baculovirus-derived human Src, 250 µM ATP and 100 µM sodium orthovanadate. Filters were washed briefly with kinase buffer alone then incubated in stripping buffer (62.5 mM Tris-HCl pH7.0, 2% SDS, 100 mM 2-β-mercaptoethanol) at 50 °C for 30 minutes to remove possible associated phosphoproteins, including Src itself or Sf9 cell derived proteins, which might interfere with the screening. Tyrosine phosphorylated proteins were detected with an anti-phosphotyrosine monoclonal antibody, 4G10, using standard immunoblotting methodology. Positive primary clones were isolated, eluted into SM buffer (0.1 M NaCl, 8 mM MgSO_{4•}7H20, 50 mM Tris 7.5, 0.01% gelatin) and purified through 2 additional rounds of phosphorylation screening. pBluescript phagemids of purified clones were excised from the λZAP clones into SOLR cells using ExAssist helper phage according to the manufacturer's instructions (Stratagene).

### Lambda plaque screening and Northern hybridization

~360,000 NCI H460 λZAP library plaques were plated onto NCZYM plates and lifted onto nitrocellulose filters. Filters were denatured (0.5M NaOH, 1.5M NaCl), neutralized (0.5M Tris 7.4, 1.5M NaCl), baked at 80C and pre-hybridized in hybridization buffer (5X SSPE, 10X Denhardts, 2% SDS, 50% Formamide, 0.1 mg/mL Salmon Sperm DNA) for 3-6 hours. The original 1.6 kb RI/XhoI tks 107 fragment (from excised phagemid) was purified and labelled with Boehringer Mannheim's Random Primed DNA Labelling Kit and purified with Qiagen's QiaQuick PCR purification kit. Filters were probed with this labelled fragment during an overnight hybridization in fresh hybridization buffer at 42 °C followed by 3X10 minutes washes at room temperature with 2X SSC, 0.05% SDS and 2X 20' with 0.1X SSC, 0.1% SDS at 50 °C. The filters were air dried and expose to X-OMAT-AR film (Kodak) overnight with 1 intensifying screen at -80 °C. Positive plaques were isolated and purified to homogeneity through subsequent rounds of hybridization screening. pBluescript phagemids of purified clones were exised from the λZAP clones into SOLR cells as described above.

Approximately 270,000 TriplEX-Prostate library plaques (Clontech) were similarly probed with a -500 b.p. EcoRI/XbaI fragment obtained from the original tks 202 clone. This fragment was random prime labelled using the Ready to Go DNA Labelling Beads and purified with Probe Quant G-50 Micro columns (Pharmacia).

Human Multiple Tissue Northern Blots I and II (Clontech) were hybridized with the 1.6 kb EcoRI/XhoI fragment from tks 107, a 1.5 kb EcoRI fragment from tks 113, the 1.6 kb EcoRI/XhoI fragment from tks 118, and -500 b.p. EcoRI/XbaI fragment from tks 202 clone using the same labelling, hybridization and wash protocols as above. The blots were stripped by boiling in 0.5% SDS for 10 minutes and washed with 2X SSC between probes.

All molecular biology techniques and buffers were performed and prepared as described in Sambrook, et. al. 1989.

### Constructs

pCnM-107 was constructed by cloning the 4.65 kb NarI/PvuI fragment (containing the entire open reading frame [ORF] of tks 107 with about 90 n.t. of 3' untranslated region [UTR]) into the EcoRV site of pCMVneoMyc2. This creates an N-term Myc tagged, full length tks 107 under transcriptional regulation of the CMV promoter. pCnM-107N, which contains an in frame 466 amino acids deletion of the C-term of pCnM-107, was created by digestion of pCnM-107 with BstEII/XhoI, filling in with dNTP's and T4 polymerase, and reclosing with T4 DNA ligase. pCnM-107C, consisting of the C-term 477 a.a. of tks 107 fused to the Myc tag of pCnM2, was created by an in frame deletion of the BamHI/BstEII fragment of pCnM-107. PCANMyc-GrubDH/PH was generated by cloning a BamHI/XhoI PCR fragment encoding just the DH/PH domains of Grub into the BamHI/XhoI sites of pCAN-MycA. PCAN-MycA is a pCDNA3 (Invitrogen) derivative with a Myc tag inserted into the polylinker. The primers used for the PCR were CCCCGGATCCGAGCGCAAGCGAAGCAT and AACTCCTCGAGCTAGGCTGCCTGTCTCCAC (italicized nucleotides represent the BamHI or XhoI sites; underlined CTA is an engineered stop codon). PCAN-Myc-GrubDH was generated by cloning a BamHI/XhoI PCR fragment encoding just the DH domain of Grub into pCANMycA. The primers used for the PCR were CCCCGGATCCGAGCGCAAGCGAAGCAT and CACCTCGAGCTACGCTTCCACGGCCAGCAGGTC (italicized nucleotides represent the BamHI or XhoI sites; underlined CTA is an engineered stop codon).

A ~330 n.t. BamHI/NcoI PCR fragment of tks 118 was generated which fused the 5' coding region of tks118 with a BamHI site. The NcoI site is naturally found in tks 118. Primers used were: 5' primer, GCTGGGATCCGATCTATGCGCGCGAACCTG (SEQ ID NO:9) (italicized nucleotides represent the BamHI site; underlined ATG is tks 118's initiating methionine); 3' primer, TGAAAGCTGTAGTTGGCACCTGA (SEQ ID NO:10). This PCR product was used to replace the BamHI/NcoI fragment in tks 118. This effectively deletes all 5' UTR. The BamHI/HindII fragment from the resultant construct (encoding full length tks 118) was cloned into the BamHI/EcoRV site of pCnM2 to yield pCnM-118. An expression construct missing the SH3 domain of Dresh, pCnM-118ΔSH3, was created by cloning the BamHT/XcmI fragment of pCnM-118 into the BamHI/EcoRV sites of pCnM2. A construct expressing just the SH3 domain of tks118, pCnM-118SH3, was created by cloning the XcmI/Asp718 fragment of pCnM-118 into the StuI/Asp718 sites of pCnM2.

A construct expressing the SH3 domain of tks118 fused to glutathione-S-transferase (GST) was created by PCR amplification of the SH3 domain using the primers 5'CGCGAATTCCGGGCTCAGTGGGCAAGGG (SEQ ID NO:11) and GGGCTCGAGTCACTCAATGAGCTCCAC (SEQ ID NO:12) 3', digesting with EcoRI/XhoI and cloning into the same sites of pGEX-4T-3 (Pharmacia), resulting in the plasmid pGEX-118SH3.

A construct expressing the N-term of tks 113 fused to GST was created by cloning the EcoRI tks 113 fragment into pGEX-4T-3, resulting in pGEX-113a. An Asp718/XhoI fragment, encoding the C-term of tks113, was deleted from pGEX-113a to yield pGEX-113c.

### 5' RACE-PCR

A modification of 5' RACE PCR was carried out in order to extend the 5' end of Tks202. First strand cDNA was made from human pancreas mRNA by Oligo DT primed reverse transcription. Second strand was primed by the oligo (ML2G) AAGTGGCAACAGAGATAACGCGTACCGGG. The 3 G's at the 3' end of ML2G base pairs with runs of non-specific C's added by reverse transcriptase at the end of transcripts. Tks 202 5' RACE PCR reaction was performed with a tks202 specific primer of sequence GGCTGGTTCGGCCACTTGAGGG and ML2G using the GC-Rich PCR kit (Roche Molecular Biochemicals). The PCR reaction was separated on an agarose gel, transferred to nitrocellulose, and hybridized (as described above) with a random primed (Ready to Go Beads from Pharmacia) labelled 550 nt DNA fragment from the 5' end of tks202-17. An approximately 500 bp labelled band was excised and cloned into pCR 2.1 Topo using the Topo TA cloning kit (Invitrogen). Colonies with the desired inserts were identified by colony hybridization using the same tks202-17 probe. DNA from these colonies were prepared and sequenced.

### Cell cultures, and DNA transfection

293, NIH3T3, and an NIH3T3 cell line stably expressing the activated chicken Src allele Y527F (NIH3T3-Y527F)were grown in Dulbecco's modified Eagle's Medium (DMEM) containing 10% FCS and antibiotics. Confluent 293 cells were split 1:10 or NIH3T3's were split 1:5 the day prior to DNA transfection. Cells were transfected with 0.1-5 µg of DNA for 4-20 hrs using Lipofectamine (Gibco) or Effectene (Qiagen) according to manufacturers' instructions. Transfected cells were recovered overnight in DMEM, 10% FCS, and antibiotics.

### Antibodies

Production of GST-DreshSH3 and GST-113c fusion proteins were induced in bacteria and purified over Glutathione-agarose beads (Pharmacia). Cell pellets from a 400mL induction were resupended in 30 mL 10 mM Tris 8.0, 150 mM NaCl, 1 mM EDTA (STE) with 100 mg/mL lysozyme, 10 µg/mL aprotinin, 20 µM leupeptin and 100 µM (PMSF) and sonicated to lyse cells. Lysates were centrifuged for 15' at 17K g's. 10 mL of PBS was added to the supernatant and Tween 20 was added to a final concentration of 0.1%. This was applied onto a Glutathione-agarose column and washed extensively with PBS with 0.1% Tween 20. Fusion proteins were eluted with 75 mM Hepes 7.5, 150 mM NaCl, 200mM glutathione, 5 mM DTT and 0.1% octylglucoside. Eluate was dialyzed against PBS.

Polyclonal antibodies were raised by immunizing rabbits with the two fusion proteins (Animal Pharm). Antibodies were precipitated with (NH₄)₂SO₄, resuspended in PBS and passed over a column with coupled GST to remove GST specific antibodies. The resultant eluate were affinity purified by passing over columns containing the GST fusion proteins coupled to CN-Br activated Sepharose 4B (Pharmacia).

### Cell lysis, immunoprecipitation, immunoblotting

Cells destined for lysis were washed in (PBS + Na3VO4) and lysed in RIPA lysis buffer (20 mM Tris 7.5, 150 mM NaCl, 1% TX100, 1% Deoxycholate, 0.1 % SDS) or HNTG (1.5mM MgCl₂, 150mM NaCl, 50mM Hepes 7.5, 10% glycerol, 1% TX100, 1mM EGTA) containing 100 µM Na₃VO₄, 10mM NaF, 0.5-2.0mM DTT, 10µg/ml aprotinin, 20µM leupeptin and 100µM phenylmethylsulfonylflouride (PMSF). Extracts were clarified by centrifugation at 16,000 g's for 10 minutes.

Approximately 0.2-5.0 mg of total proteins were immunoprecipitated by incubation with 2-20µg of 9E10 antibody or 12CA5 and 20uL of Protein A/G (Santa Cruz Biotechnology) at 4°C for 4 hours. Immunocomplexes were washed 4X with lysis buffer, resuspended in 45uL SDS sample buffer (80mM Tris 6.8, 2%SDS, 10% glycerol, 1.25% bromophenol blue and 280 mM B-mercaptoethanol), and heated at 95°C for 3-5 minutes. Samples were subjected to SDS-PAGE and immunoblotted onto nitrocellulose membranes (MSI) using a Millipore semi-dry blotting apparatus.

Filters were blocked in TBST (25 mM Tris 7.5, 150mM NaCl and 0.1 % Tween 20) with 3% BSA or 2% non-fat dry milk for 1 hour at room temperature. Filters were incubated in blocking buffer with primary antibodies for 1 hours using the following dilutions: 12CA5, 9E10, and 4G10 1:5000; α-cst1, 1:200; α-118SH3 (α5033) and α-tks 118 (α5029), 1:1000. Filters were washed 3x 10 minutes with TBST and incubated in donkey anti-rabbit HRP (Amersham) or sheep anti-mouse HRP (Amersham) at 1:10000 in blocking buffer for 1 hour. Filters were washed as previous and the protein bands detected using enhanced chemiluminescence (SuperSignal, Pierce) in conjunction with Fuji RX, Film.

### Cell Staining

NIH3T3 or NIH3T3-Y527F cells were split 1:5 from a confluent dish onto coverslips. Cells were transfected with various plasmids as described above and in the main text. Cells on coverslips were fixed with 3% para-formaldehyde in PBS and permeabilized with 0.2% TX100 in PBS. Cells were stained with various combinations of antibodies diluted in 0.2% TX100, 0.2% BSA in PBST. Primary antibodies used were diluted 1/50. Secondary antibodies were also diluted 1:50 and were Rabbit-TexRed (Immuno Research) and Mouse-Oregon-Green (Molecular Probes). Cells were also stained with Bis-benzimide to stain the nucleus.

### JNK Kinase Assay

293 cells were transfected with pCAN-HA-JNK and various expression plasmids. Cells were lysed in HNTG and 0.2 mg were immunoprecipitated with 12CA5 for 4 hrs. Immunoprecipitates were washed 3X with lysis buffer and 2X with Kinase Buffer (25mM Hepes7.6, 20mM MgCl₂, 2mM DTT, 100µM Na₃VO₄, 1mM PMSF). 1/2 of each immunoprecipitate was run on acrylamide gel and Western blotted for HA-JNK expression level. The remainder was resuspended in 30µL kinase buffer with 20µM ATP, 5µCi γP³²ATP, and 5µg GST-Jun 1-223 and incubated at 30°C for 30 minutes. Reactions were stopped with 7µL 5x Laemmli sample buffer, boiled, separated on a 10% SDS-PAGE gel, dried, and exposed to film. The GST-Jun bands were excised and counted on a Scintillation counter.

### 2-hybrid Screen

The BamHI fragment encoding the N-term domain of Grub from pCnM-107N was cloned into the BamHI site of pEG202 to produce pEG-107N. This plasmid encodes the N-term of Grub fused to the DNA binding domain LexA. This bait construct was introduced into the S. cervisiae strain EGY48 producing a resulting strain. EGY48 contains the LEU2 and β-galactosidase genes under the control of a synthetic promoter with LexA binding sites. A Hela cDNA expression library in the yeast expression vector pJG45 was transformed into the resultant strain (Gyuris, J., Golemis, E., Chertkov, H., and Brent, R. (1993) Cell 75, 791-803). Approximately 1.8 X 106 transformants were screened for interacting clones. 6 independent cDNA's were found to be identical to a previously isolated gene Snapin (Ilardi, et. al. Nature Neuro. (1999) 2, 119-124). 2 independent overlapping clones were found to be the human orthologue of the rat RACK1 (Ron, D., et. al. P.N.A.S. (1994) 91, 839-843).

### CONCLUSION

One skilled in the art would readily appreciate that the present invention is well adapted to carry out the objects and obtain the ends and advantages mentioned, as well as those inherent therein. The molecular complexes and the methods, procedures, treatments, molecules, specific compounds described herein are presently representative of preferred embodiments, are exemplary, and are not intended as limitations on the scope of the invention. It will be readily apparent to one skilled in the art that varying substitutions and modifications may be made to the invention disclosed herein without departing from the scope and spirit of the invention.

All patents and publications mentioned in the specification are indicative of the levels of those skilled in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising," "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

In addition, where features or aspects of the invention are described in terms of Markush groups, those skilled in the art will recognize that the invention is also thereby described in terms of any individual member or subgroup of members of the Markush group. For example, if X is described as selected from the group consisting of bromine, chlorine, and iodine, claims for X being bromine and claims for X being bromine and chlorine are fully described.

In view of the degeneracy of the genetic code, other combinations of nucleic acids also encode the claimed peptides and proteins of the invention. For example, all four nucleic acid sequences GCT, GCC, GCA, and GCG encode the amino acide alanine. Therefore, if for an amino acid there exists an average of three codons, a polypeptide of 100 amino acids in length will, on average, be encoded by 3100, or 5 x 1047, nucleic acid sequences. Thus, a nucleic acid sequence can be modified to form a second nucleic acid sequence, encoding the same polypeptide as endoded by the first nucleic acid sequences, using routine procedures and without undue experimentation. Thus, all possible nucleic acids that encode the claimed peptides and proteins are also fully described herein, as if all were written out in full taking into account the codon usage, especially that preferred in humans. Furthermore, changes in the amino acid sequences of polypeptides, or in the corresponding nucleic acid sequence encoding such polypeptide, may be designed or selected to take place in an area of the sequence where the significant activity of the polypeptide remains unchanged. For example, an amino acid change may take place within a β-turn, away from the active site of the polypeptide. Also changes such as deletions (e.g. removal of a segment of the polypeptide, or in the corresponding nucleic acid sequence encoding such polypeptide, which does not affect the active site) and additions (e.g. addition of more amino acids to the polypeptide sequence without affecting the function of the active site, such as the formation of GST-fusion proteins, or additions in the corresponding nucleic acid sequence encoding such polypeptide without affecting the function of the active site) are also within the scope of the present invention. Such changes to the polypeptides can be performed by those with ordinary skill in the art using routine procedures and without undue experimentation. Thus, all possible nucleic and/or amino acid sequences that can readily be determined not to affect a significant activity of the peptide or protein of the invention are also fully described herein.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims.

## Claims

1. An isolated, enriched, or purified Grub polypeptide comprising:
(a) the amino acid sequence of SEQ ID NO: 5 or a functional derivative thereof; or
(b) an amino acid sequence having at least 90% sequence identity to SEQ ID NO: 5 or a functional derivative thereof.

2. The Grub polypeptide of claim 1, wherein said polypeptide is isolated, enriched, or purified from a mammal.

3. The Grub polypeptide of claim 2, wherein said mammal is a human.

4. An isolated, enriched, or purified nucleic acid molecule encoding a Grub polypeptide of any of claims 1 to 3 or the complement thereof.

5. The nucleic acid molecule of claim 4 having the sequence of SEQ ID NO: 1 or the complement thereof.

6. The nucleic acid molecule of claim 4 or 5, further comprising a vector or promoter effective to initiate transcription in a host cell.

7. An antibody or antibody fragment having specific binding affinity to a Grub polypeptide of any of claims 1 to 3.

8. A hybridoma producing an antibody or antibody fragment of claim 7.

9. A method for identifying a substance that modulates the activity of a Grub polypeptide of any of claims 1 to 3, the method comprising:
(a) contacting the Grub polypeptide with a test substance;
(b) measuring the activity of said Grub polypeptide; and
(c) determining whether said substance modulates the activity of said Grub polypeptide.

10. The method of claim 9, wherein the activity of the Grub polypeptide comprises the interaction between said Grub polypeptide and Src.

11. A method for identifying a substance that modulates the activity of a Grub polypeptide of any of claims 1 to 3 in a cell, the method comprising:
(a) expressing a nucleic acid molecule encoding the Grub polypeptide in a cell;
(b) adding a test substance to said cell; and
(c) monitoring a change in cell phenotype or the interaction between said Grub polypeptide and a natural binding partner.

12. The method of claim 11, wherein the natural binding partner is Src.

13. Use of a substance that modulates the activity of a Grub polypeptide of any of claims 1 to 3 for the manufacture of a medicament for the treatment of a disease selected from the group consisting of rheumatoid arthritis, atherosclerosis, autoimmune disorders, organ transplantation, myocardial infarction, cardiomyopathies, stroke, renal failure, oxidative stress-related neurodegenerative disorders, and cancer.
